(19) Europäisches Patentamt
European Patent Office
Office européen des brevets

(11) Publication number: **0 313 397 B1**

# (12) EUROPEAN PATENT SPECIFICATION

(45) Date of publication of patent specification: **02.06.93**

(51) Int. Cl.5: **C07D 403/06**, C07D 413/06, C07D 405/14, C07D 409/14, A61K 31/40, A61K 31/41, A61K 31/415, A61K 31/42

(21) Application number: **88309943.4**

(22) Date of filing: **21.10.88**

## (54) Therapeutic heterocyclic compounds.

(30) Priority: **23.10.87 GB 8724912**

(43) Date of publication of application: **26.04.89 Bulletin 89/17**

(45) Publication of the grant of the patent: **02.06.93 Bulletin 93/22**

(84) Designated Contracting States: **AT BE CH DE ES FR GB GR IT LI LU NL SE**

(56) References cited:
**EP-A- 0 225 726**
**GB-A- 2 185 020**

(73) Proprietor: **THE WELLCOME FOUNDATION LIMITED**
**Unicorn House 160 Euston Road**
**London NW1 2BP(GB)**

(72) Inventor: **Robertson, Alan Duncan**
**Langley Court**
**Beckenham Kent(GB)**
Inventor: **Martin, Graeme Richard**
**Langley Court**
**Beckenham Kent(GB)**
Inventor: **Buckingham, Janet Susan**
**Langley Court**
**Beckenham Kent(GB)**

(74) Representative: **Garrett, Michael et al**
**The Wellcome Research Laboratories Group Patents and Agreements Langley Court**
**Beckenham Kent BR3 3BS (GB)**

EP 0 313 397 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is concerned with new chemical compounds, their preparation, pharmaceutical formulations containing them and their use in medicine, particularly in the treatment of migraine.

Receptors which mediate the actions of 5-hydroxytryptamine (5-HT) have been identified in mammals in both the periphery and the brain. According to the classification and nomenclature proposed in a recent article (Bradley et al, Neuropharmac., 25, 563 (1986)), these receptors may be classified into three main types, viz. "5-$HT_1$-like", 5-$HT_2$ and 5-$HT_3$. Various classes of compounds have previously been proposed as 5-HT agonists or antagonists for therapeutic use, but these have not always been specific to a particular type of 5-HT receptor. We have now discovered a novel class of 5-HT agonists which are specific to a particular type of "5-$HT_1$-like" receptor and will be effective therapeutic agents for the treatment of clinical conditions in which a selective agonist for this type of receptor is indicated. For example, the receptor in question mediates vasoconstriction in the carotid vascular bed and thereby modifies blood flow therein; the compounds of the invention will therefore be beneficial in the treatment or prophylaxis of conditions wherein vasoconstriction in this bed is indicated, for example, migraine, a condition associated with excessive dilation of the carotid vasculature. However, it is within the scope of the present invention that the target tissue may be any tissue wherein action is mediated by "5-$HT_1$-like" receptors of the type referred to above.

UK Patent Specification 2185020 describes a series of 5-substituted 3-aminoalkylindoles having potential anti-migraine activity wherein the 5-substituent consists of a phenyl group joined to the indole moiety by an amidic or sulphonylamino linkage. European Patent Specification 0225726 describes a further series of 5-substituted 3-aminoalkylindoles having potential anti-migraine activity wherein the 5-substituent is typically carboximidamide or a heterocyclic moiety.

We have now unexpectedly found that 5-substituted 3-aminoalkylindoles wherein the 5-substituent consists of a phenyl group linked to the indole moiety via a heterocyclic group are surprisingly effective selective agonists for the particular type of "5-$HT_1$-like" receptor implicated in the aetiology of migraine.

According to a first aspect of the present invention, there is provided a compound of formula (I)

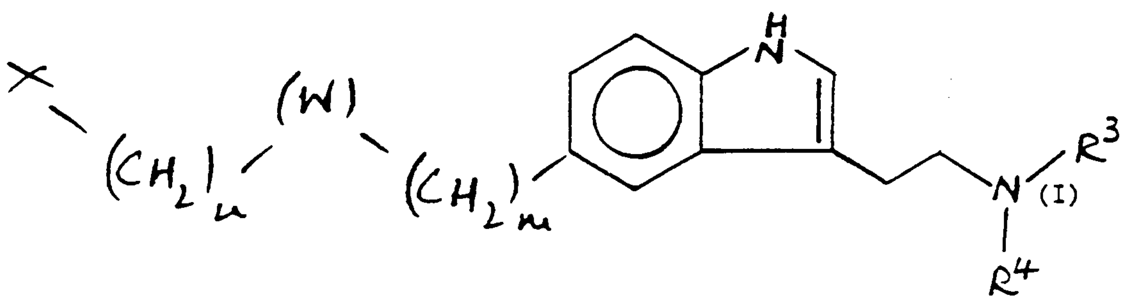

wherein

$R^3$ and $R^4$ are independently selected from hydrogen, $C_{1-6}$ alkyl (including cycloalkyl) and aryl (wherein the alkyl or aryl group, which latter includes benzyl, is optionally substituted by one or more atoms or groups independently selected from halogen, $C_{1-4}$ alkyl and aryl), provided $R^3 \neq$ benzyl or substituted benzyl when $R^4$ = H;

m is an integer of from 0 to 2;

n is an integer of from 0 to 3;

(W) is a group of formula (i), (ii), (iii), or (iv)

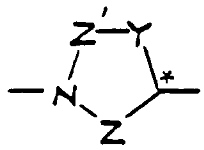 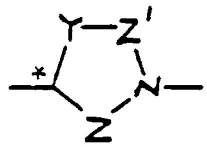 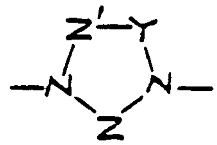 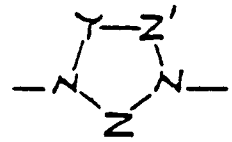

(i) (ii) (iii) (iv)

wherein Y is selected from oxygen, methylene and >N-$R^5$, where $R^5$ is hydrogen, $C_{1-4}$ alkyl, or benzyl, Z and Z' are independently selected from >C=O, >C=S and methylene, and the chiral centre * in formula (i) or (ii) is in its (S) or (R) form or is a mixture thereof in any proportions;

X is a group selected from

aryl
xanthenyl
dibenzofuranyl
heteroaryl

which group is optionally substituted by an atom or group selected from

aryl
$C_{1-4}$ alkoxy
aryloxy
$C_{1-6}$ alkylsulphonyl
arylsulphonyl
halogen
cyano
carbamoyl
$C_{1-6}$ alkylcarbamoyl
$(C_{1-6})_2$ dialkylcarbamoyl
arylcarbamoyl
$C_{2-7}$ alkanoylamino
aroylamino
$C_{1-6}$ alkylsulphamino

the aryl group(s) of aryl-containing substituents being optionally substituted by halogen or $C_{1-4}$ alkoxy; and salts and solvates thereof.

Compounds of formula (I) having particularly desirable pharmacological properties for the treatment or prophylaxis of migraine include those in which (W) is (i) wherein Y = 0, $CH_2$, or $NR^5$, Z = CO or $CH_2$ and Z' = CO, (W) is (ii) wherein Y = $CH_2$ or $NR^5$ and Z = Z' = CO, (W) is (iii) wherein Y = $CH_2$ or $NR^5$, Z = CO and Z' = CO or $CH_2$, or (W) is (iv) wherein Y = $CH_2$, Z = CO and Z' = CO or $CH_2$, and X is a phenyl ring substituted by an alkylcarbamoyl, alkanoylamino, or alkylsulphamino group.

Particularly preferred compounds of formula (I) include 2-{5-[1-(3-methylaminocarbonylbenzyl)-2,5-dioxoimidazolidin-4-ylmethyl]-1H-indol-3-yl} ethylamine, N,N-dimethyl-2-[5-{1-[2-(4-acetylaminophenyl)-ethyl]-2,5-dioxoimidazolidin-4-ylmethyl}-1H-indol-3-yl]ethylamine, N-methyl-2-{5-[1-(3-methylaminocarbonylbenzyl)-2,5-dioxoimidazolidin-4-ylmethyl]-1H-indol-3-yl}ethylamine, 2-{5-[1-(3-acetylaminobenzyl)-2,5-dioxoimidazolidin-4-ylmethyl]-1H-indol-3-yl}ethylamine and 2-{5-[1-(3-methylsulphonylaminobenzyl)-2,5-dioxoimidazolidin-4-ylmethyl]-1H-indol-3-yl}ethylamine; in particular, salts thereof.

Physiologically acceptable salts of the present compounds are particularly suitable for medical purposes because of their greater aqueous solubility relative to the parent, i.e. basic, compounds. Such salts must clearly have a physiologically acceptable anion. Suitable physiologically acceptable salts include those derived from acetic, hydrochloric, hydrobromic, phosphoric, malic, maleic, fumaric, citric, sulphuric, lactic, or tartaric acid, The maleate and chloride salts are particularly preferred for medical purposes. Salts having a non-physiologically acceptable anion are within the scope of the invention as useful intermediates for the preparation of physiologically acceptable salts and/or for use in non-therapeutic, for example, in vitro, situations.

According to a second aspect of the present invention, there is provided a compound of formula (I) or a physiologically acceptable salt or solvate thereof for use as a selective "5-$HT_1$-like" receptor agonist, for

example, as a vasoconstrictor, in a method of treatment of the human or animal body by therapy, such as the treatment or prophylaxis of migraine. As indicated earlier, however, target organs for the present compounds other than the carotid vasculature are within the scope of the present invention.

The amount of a compound of formula (I), or a salt or solvate thereof, which is required to achieve the desired biological effect will depend on a number of factors such as the specific compound, the use for which it is intended, the means of administration, and the recipient. A typical daily dose for the treatment of migraine may be expected to lie in the range 0.05 to 30mg per kilogram body weight. Unit doses may contain from 1 to 500mg of a compound of formula (I), for example, ampoules for injection may contain from 1 to 50mg and orally administrable unit dose formulations such as tablets or capsules may contain from 1 to 500mg. Such unit doses may be administered one or more times a day, separately or in multiples thereof. An intravenous dose may be expected to lie in the range 0.05 to 50mg/kg and would typically be administered as an infusion of from 0.0005 to 2.0mg per kilogram per minute. Infusion solutions suitable for this purpose may contain from 0.01 to 10mg/ml.

When the active compound is a salt or solvate of a compound of formula (I), the dose is based on the cation (for salts) or the unsolvated compound.

Hereinafter references to "compound(s) of formula (I)" will be understood to include physiologically acceptable salts and solvates thereof.

According to a third aspect of the present invention, therefore, there are provided pharmaceutical compositions comprising, as active ingredient, at least one compound of formula (I) and/or a pharmaceutical carrier salt or solvate thereof together with at least one pharmacologically acceptable or excipient. These pharmaceutical compositions may be used in the treatment or prophylaxis of clinical conditions for which a selective "5-$HT_1$-like" receptor agonist of the present type is indicated, e.g. migraine. The carrier mut be pharmaceutically acceptable to the recipient and must be compatible with, i.e. not have a deleterious effect upon, the other ingredients in the composition. The carrier may be a solid or liquid and is preferably formulated with at least one compound of formula (I) as a unit dose formulation, for example, a tablet which may contain from 0.05 to 95% by weight of the active ingredient. If desired, other pharmacologically active ingredients may also be incorporated in the pharmaceutical compositions of the invention.

Possible formulations include those suitable for oral, rectal, topical (for example, buccal, such as sub-lingual), or parenteral (for example, subcutaneous, intramuscular, or intravenous) administration. The most suitable means of administration for a particular patient will depend on the nature and severity of the condition being treated and on the nature of the active compound, but, where possible, oral administration is preferred.

Formulations suitable for oral administration may be provided as discrete units, such as tablets, capsules, cachets, or lozenges, each containing a predetermined amount of the active compound; as powders or granules; as solutions or suspensions in aqueous or non-aqueous liquids; or as oil-in-water or water-in-oil emulsions.

Formulations suitable for buccal administration include lozenges comprising the active compound and, typically, a flavoured base, such as sugar and acacia or tragacanth, and pastilles comprising the active compound in an inert base, such as gelatin and glycerin or sucrose and acacia.

Formulations suitable for parenteral administration typically comprise sterile aqueous solutions containing a predetermined concentration of the active compound; the solution is preferably isotonic with the blood of the intended recipient. Although such solutions are preferably administered intravenously, they may also by administered by subcutaneous or intramuscular injection.

Formulations suitable for rectal administration are preferably provided as unit-dose suppositories comprising the active ingredient and one or more solid carriers forming the suppository base, for example, cocoa butter.

Formulations suitable for topical application include ointments, creams, lotions, pastes, gels, sprays, aerosols and oils. Suitable carriers for such formulations include petroleum jelly, lanolin, polyethylene glycols, alcohols, and combinations thereof. The active ingredient is typically present in such formations at a concentration of from 0.1 to 15% w/w.

The formulations of the invention may be prepared by any suitable method, typically by uniformly and intimately admixing the active compound(s) with liquids or finely divided solid carriers, or both, in the required proportions and then, if necessary, shaping the resulting mixture into the desired shape.

For example, a tablet may be prepared by compressing an intimate mixture comprising a powder or granules of the active ingredient and one or more optional ingredients, such as a binder, lubricant, inert diluent, or surface active dispersing agent, or by moulding an intimate mixture of powdered active ingredient and inert liquid diluent.

Aqueous solutions for parenteral administration are typically prepared by dissolving the active compound in sufficient water to give the desired concentration and then rendering the resulting solution sterile and isotonic.

Thus, according to a fourth aspect of the present invention, there is provided the use of a compound of formula (I) in the preparation of a pharmaceutical formation for the treatment or prophylaxis of clinical conditions in which a selective agonist for the particular type of "5-$HT_1$-like" receptor described herein is indicated, for example, migraine.

According to a fifth aspect, there is provided a method of treating or preventing clinical conditions in which a selective agonist for the particular type of "5-$HT_1$-like" receptor described herein is indicated, for example, migraine, using a compound of formula (I) or a pharmaceutical formulation containing such a compound.

According to a sixth aspect of the invention, compounds of formula (I) may be prepared by any suitable process, for example, by reacting a compound of formula (II)

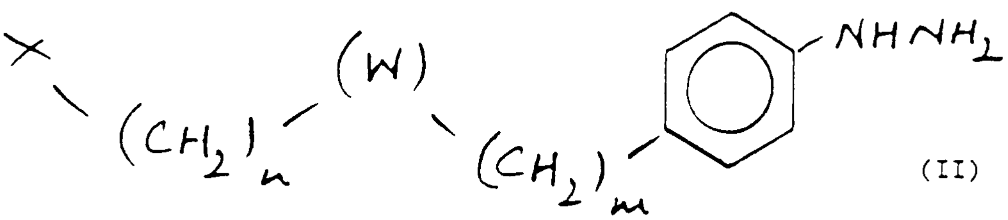

(II)

wherein n, m, (W) and X are as hereinbefore defined, with a compound of formula (III)

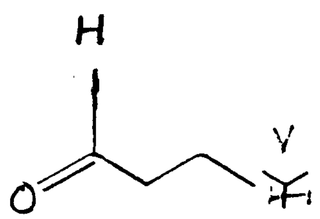

(III)

or a carbonyl-protected form thereof, such as the dimethyl acetal, wherein V is either $-CH_2L$ wherein L is (a) a suitable leaving group, such as chloro, and the initial product is aminated, (b) a protected form of amino group, for example, phthalimido, and the initial product is deprotected, (c) a nitro group and the initial product is reduced, or (d) a group $-NR^3R^4$ wherein $R^3$ and $R^4$ are as hereinbefore defined other than hydrogen, or V is cyano and the initial product is reduced. Methods by which the initial products may be aminated, deprotected, or reduced are well known to those skilled in the art.

The reaction of compounds (II) and (III) to give a compound of formula (I) may be carried out in a single step (Fischer indole synthesis) or by an initial non-cyclising step at a lower temperature to give a compound of formula

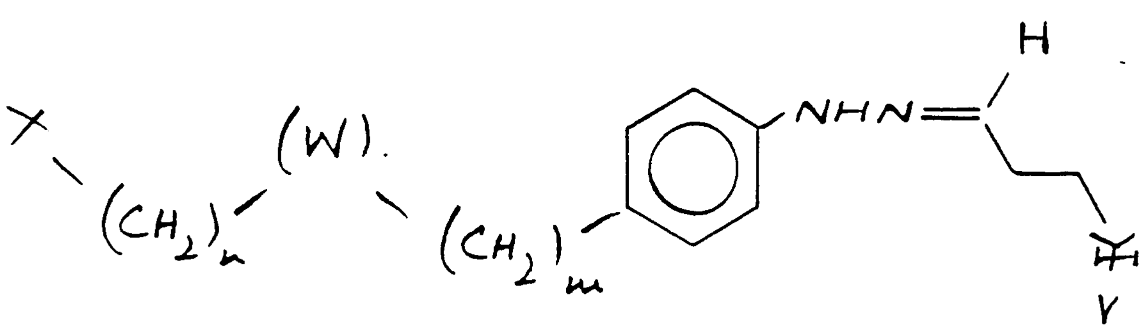

wherein (W), X, V, m and n are as hereinbefore defined, followed by cyclisation using a suitable reagent, such as a polyphosphate ester, to give a compound of formula (I).

Standard N-alkylation/arylation methods may be used to convert compounds of formula (I) wherein $R^3$ and/or $R^4$ are hydrogen to corresponding compounds wherein $R^3$ and/or $R^4$ are alkyl or aryl as hereinbefore defined.

Compounds of formula (I) wherein $R^3 = R^4 = C_{1-6}$ alkyl or aryl may be prepared from the corresponding compound wherein $R^3 = R^4 = H$ by methods of N-alkylation or N-arylation well known to those skilled in the art, for example, by treatment with the appropriate aldehyde in the presence of a reducing agent, for example, sodium cyanoborohydride.

Compounds of formula (I) wherein $R^3$ or $R^4 = C_{1-6}$ alkyl or aryl may be prepared from the corresponding compound wherein $R^3 = R^4 = H$ by N-benzylation using benzaldehyde and a suitable reducing agent, for example, sodium borohydride, followed by N-alkylation or N-arylation using a suitable agent, such as dimethyl sulphate, typically under basic conditions, for example, anhy. $K_2CO_3$/DMF, and finally debenzylation by catalytic hydrogenation.

Hydrazines of formula (II) may be prepared from the corresponding anilines of formula (IV)

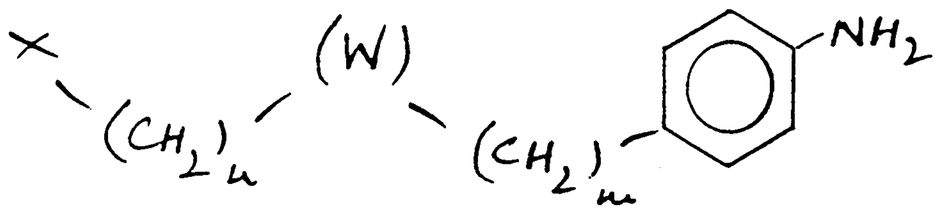

(IV)

wherein m, n, (W) and X are as hereinbefore defined, by diazotisation followed by reduction. Diazotisation is typically carried out using sodium nitrite/c.HCl and the resulting diazo product reduced <u>in situ</u> using, for example, tin(II) chloride/c.HCl.

Anilines of formula (IV) may be prepared by reduction of the corresponding nitro compounds of formula (V)

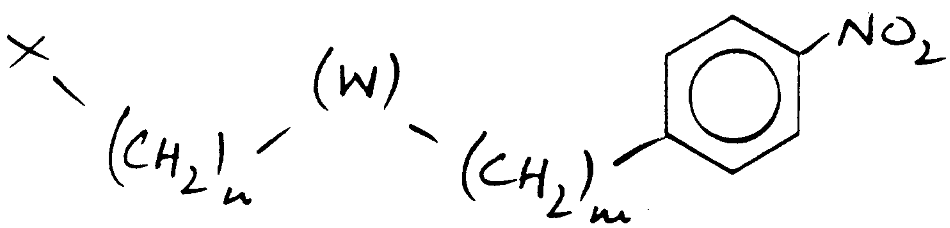

(V)

wherein m, n, (W) and X are as hereinbefore defined, typically by catalytic hydrogenation or using tin(II) chloride.

Nitro compounds of formula (V) may be prepared by

(a) reacting a compound of formula (VI)

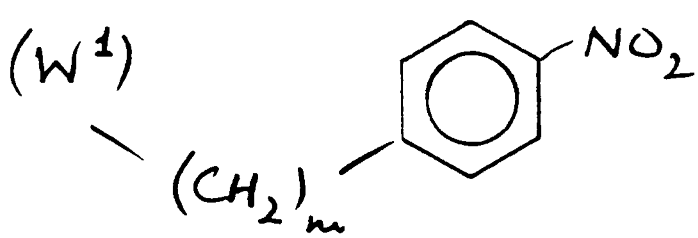

(VI)

wherein m is as hereinbefore defined and ($W^1$) is

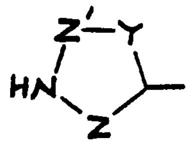,

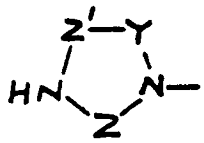, or 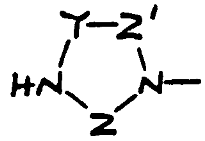,

wherein Y, Z and Z' are as hereinbefore defined, with a compound of formula (VII)

$X-(CH_2)_n-M$ (VII)

wherein n and X are as hereinbefore defined and M is a suitable leaving group, such as chloro, bromo, or hydroxy, typically under basic conditions, for example, anhy. $K_2CO_3$/DMF or P-TBD/DCM (M = chloro or bromo) or DEAD/$Ph_3P$/DME (M = hydroxy). The latter conditions are suitable for the preparation of compounds of formula (V) wherein (W) is

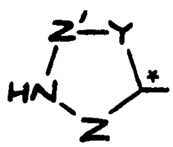

where * is a chiral centre.

Alternatively, a compound of formula (VI) wherein m is as hereinbefore defined and ($W^1$) is

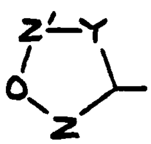, 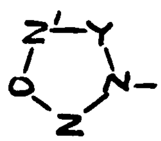, or 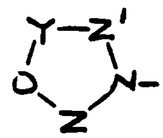,

wherein Y, Z and Z′ are as hereinbefore defined, may be reacted with a compound of formula (VII) wherein n and X are as hereinbefore defined and M is amino, typically in an aprotic solvent, followed by treatment with, for example, acetyl chloride;

(b) reacting a compound of formula (VIII)

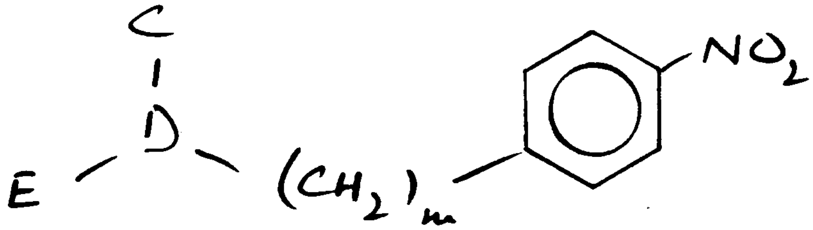

(VIII)

wherein m is as hereinbefore defined, D = CH or N, and C and E are as defined below, with a compound of formula (IX)

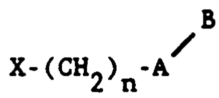 (IX)

wherein n and X are as hereinbefore defined and A and B are atoms or groups capable of reacting with groups E and C respectively to form a heterocyclic group

$-(\overline{A-B-C-D-E})-$,

or reacting a compound of formula (VIIIA)

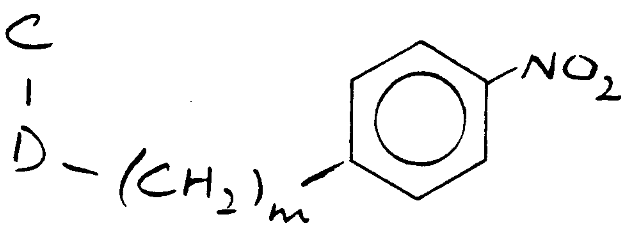 (VIIIA)

wherein m is as hereinbefore defined and C and D are as defined below, with a compound of formula (IXA)

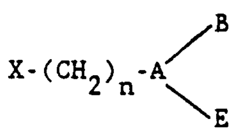 (IXA)

wherein n is as hereinbefore defined, A = CH or N, and B and E are groups capable of reacting with atoms or groups C and D to form a heterocyclic group

$-(\overline{A-B-C-D-E})-$,

thereby forming a compound of formula (V)'

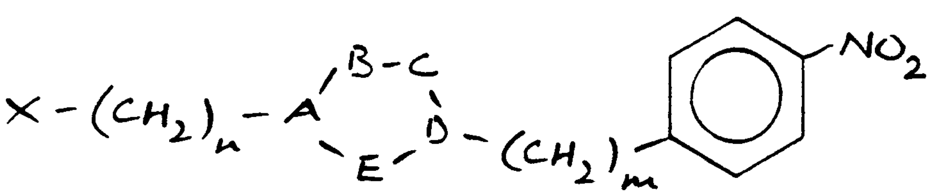 (V)'

wherein m, n and X are as hereinbefore defined and

$-\overline{A-B-C-D-E}$

is the same as (W) in compounds of formula (V). For the preparation from (VIII) and (IX) of compounds of formula (V)' wherein A = N, B = CO, C = O or NH, and E = CO, -A-B is typically -N=C=O, C is hydroxy or amino, and E is carboxy or carboalkoxy; for the preparation from (VIIIA) and (IXA) of compounds of formula (V)' wherein B = O or NH, C = CO, D = N, and E = CO, -D-C is typically -N=C=O, B is hydroxy or amino, and E is carboxy or carboalkoxy.

(c) reacting a compound of formula (X)

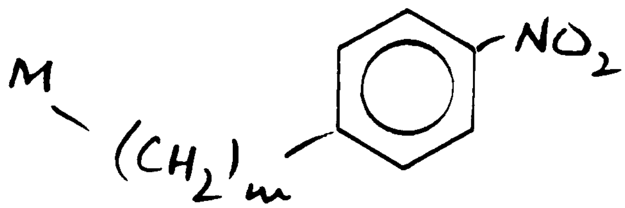

(X)

wherein m is as hereinbefore defined and M is a suitable leaving group, such as chloro, bromo, or hydroxy, with a compound of formula (XI)

$X-(CH_2)_n-(W^2)$ (XI)

wherein n and X are as hereinbefore defined and ($W^2$) is

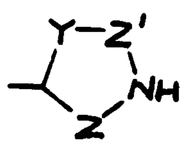

,

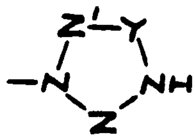

, or

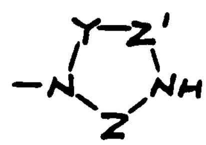

,

wherein Y, Z and Z′ are as hereinbefore defined, typically under basic conditions, for example, anhy. $K_2CO_3$/DMF or P-TBD/DCM (M = chloro or bromo) or DEAD/$Ph_3P$/DME (M = hydroxy). The latter conditions are suitable for the preparation of compounds of formula (V) wherein ($W^2$) is

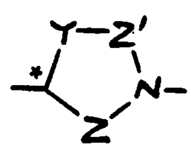

where * is a chiral centre.

Alternatively, a compound of formula (X) wherein m is as hereinbefore defined and M is amino, may be reacted with a compound of formula (XI) wherein n and X are as hereinbefore defined and ($W^2$) is

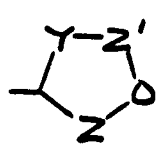

,

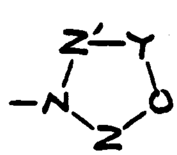

, or

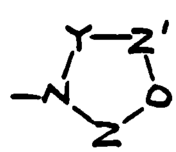

,

wherein Y, Z and Z′ are as hereinbefore defined, typically in a aprotic solvent, followed by treatment with, for example, acetyl chloride; or

(d) cyclising a compound of formula (XII)

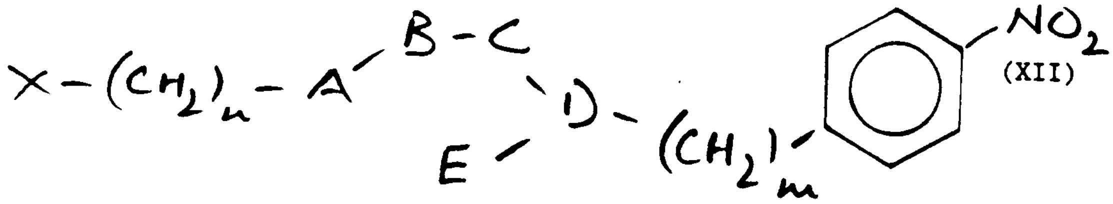

(XII)

or (XIIA)

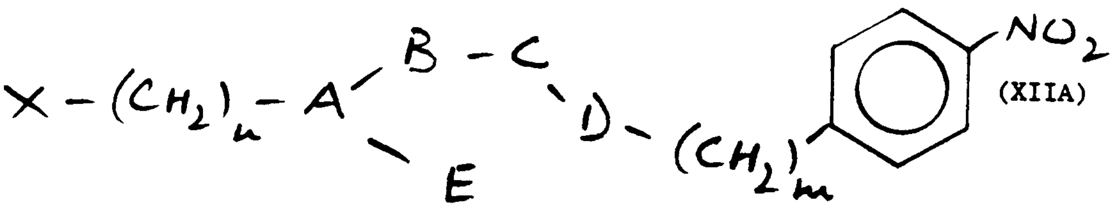

(XIIA)

wherein m, n, A, B, C, D, E and X are as hereinbefore defined, typically in the presence of acid, to form a compound of formula (V)′ wherein m, n and X are as hereinbefore defined and

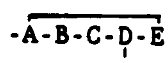

is the same as (W) in compounds of formula (V). This method is suitable for the preparation of compounds of formula (V) wherein (W) is

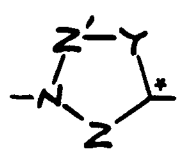

(via (XII) or

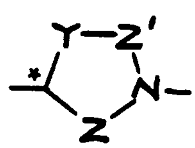

(via (XIIA) wherein * is a chiral centre. For the preparation of compounds of formula (V) wherein A = N, B = CO, C = $NR^5$, where $R^5$ is as hereinbefore defined, and E = CO, -A-B-C in compound (XII) or -B-C-D- in compound (XIIA) is typically -NH-CO-NH- and E is carboxy or carboalkoxy.

Compounds of formula (V) wherein Y = NH may be converted to compounds of formula (V) wherein Y = $NR^5$, where $R^5$ is as hereinbefore defined other than hydrogen, by treatment with a suitable alkylating or arylating agent, such as methyl iodide, typically under basic conditions, for example, anhy. $K_2CO_3$/DMF.

Compounds of formula (V) wherein Z and/or Z′ = CO may be converted to compounds of formula (IV) wherein Z and/or Z′ = $CH_2$ by suitable reduction, for example, when the carbonyl(s) is adjacent to a carbon atom, using sodium borohydride or lithium aluminium hydride (LAH) to form the alkene which is then catalytically hydrogenated. The latter step conveniently converts the nitro group of compound (V) to an amino group so that a separate reductive step is not required to prepare the aniline (IV).

Compounds of formula (VI) may be prepared by reacting a compound of formula (XIII)

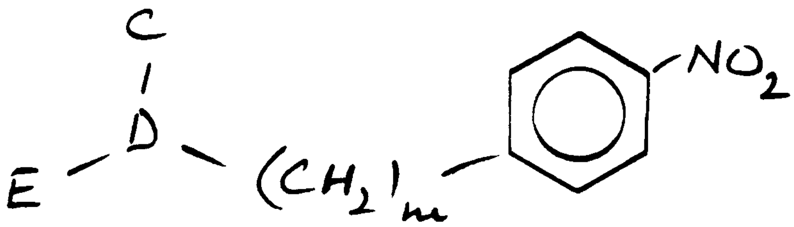

(XIII)

wherein m, C, D and E are as herinbefore defined, with a compound or anion of formula A-B wherein A and B are as hereinbefore defined, to form a compound of formula (VI)′

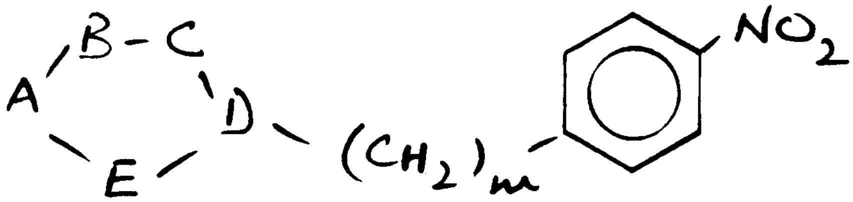

(VI)′

wherein m is as hereinbefore defined and

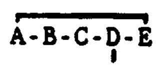

is the same as ($W^1$) is compounds of formula (VI). For the preparation of compounds of formula (VI) wherein A = NH, B = CO, C = NH and E = CO, A-B is typically cyanate anion, C is amino and E is carboxy or carboalkoxy. The reaction is carried out in a polar solvent and the resulting salt acidified _in situ_. This method is suitable for the preparation of compounds of formula (VI) wherein ($W^1$) is

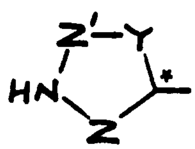

where * is a chiral centre.

Compounds of formula (VI) may also be prepared by reacting a compound of formula (X) as hereinbefore defined with a compound of formula H($W^2$) wherein ($W^2$) is as hereinbefore defined, typically in the presence of base, for example, sodium hydroxide.

Compounds of formula (XI) may be prepared by reacting a compound of formula (IXA) wherein n, A, B, E and X are as hereinbefore defined, with a compound or anion of formula C-D wherein C and D are as hereinbefore defined, to form a compound of formula (XI)′

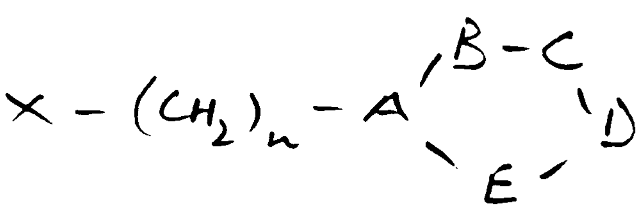

(XI)′

wherein n and X are as hereinbefore defined and

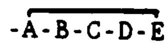

is the same as ($W^2$) in compounds of formula (XI). For the preparation of compounds of formula (XI) wherein B = NH, C = CO, D = NH and E = CO, C-D is typically cyanate anion, B is amino and E is carboxy or carboalkoxy. The reaction is carried out in a polar solvent and the resulting salt acidified in situ.

Compounds of formulae (VI)′ and (XI)′ wherein, in ($W^1$) and ($W^2$), B = E = CO, A $\overline{=}$ $\overline{O}$ and C = E = CO, D = O respectively, may be prepared by cyclisation of the appropriate dicarboxylic acid (XIV), for example, by treatment with acetyl chloride.

Compounds of formula (XII) may be prepared by reacting a compound of formula (XV)

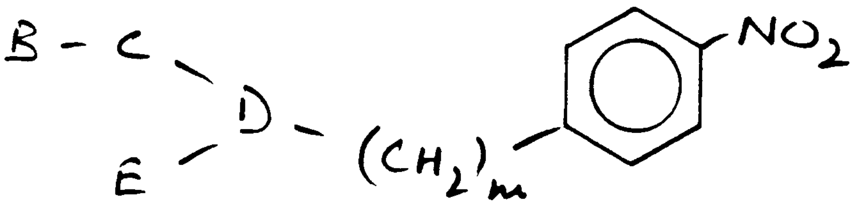

(XV)

wherein m, B, C, D and E are as hereinbefore defined, with a compound of formula (XVI)

$X-(CH_2)_n-A$ (XVI)

wherein n and X are as hereinbefore defined and A is amino, typically in an aprotic solvent.

Compounds of formula (XIIA) may be prepared by reacting a compound of formula (XVII)

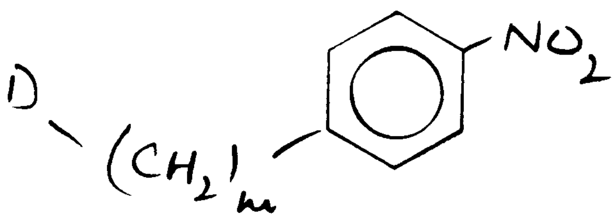

(XVII)

wherein m is as hereinbefore defined and D is amino, with a compound of formula (XVIII)

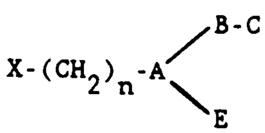

(XVIII)

wherein n, A, B, C, E and X are as herinbefore defined, typically in an aprotic solvent.

Compounds of formula (VII), (VIII), (VIIIA), (IX), (IXA), (X), (XIII), (XIV), (XV), (XVI), (XVII) and (XVIII) can be obtained commercially or may be prepared from readily available starting materials by methods known to those skilled in the art or obtainable from the chemical literature.

For a better understanding of the invention, the following Examples are given by way of illustration.

Synthetic Examples 1 to 12 are concerned with the preparation of compounds of formula (V) which may be converted to compounds of formula (I) by steps analogous to those described in Synthetic Examples 13 to 16. All intermediates in the Synthetic Examples which have a chiral centre are racemic unless otherwise indicated. All flash chromatography described in the Synthetic Examples was carried out using Merck Kieselguhr 60 (230-400 mesh ASTM).

Synthetic Example 1

Preparation of N-Methyl-3-[4-(4-nitrobenzyl)-2,5-dioxoimidazolidinylmethyl]benzamide

(a) 4-(4-Nitrobenzyl)imidazolidin-2,5-dione

A solution of p-nitrophenylalanine (4.2g, Fluka) and potassium cyanate (1.62g) in water (6ml) was refluxed for 1 hour. C.HCl (3ml) was added and the mixture refluxed for a further 10 minutes, then cooled, diluted with water and filtered. The residue was washed with water and dried to give the desired product as a straw-coloured solid (3.85g). The 200MHz $^1H$ NMR was consistent with the proposed structure.

A sample was crystallised from ethanol to give pale cream needles, mp >235 °C.

(b) 3-Chloromethyl-N-methylbenzamide

A solution of 3-chloromethylbenzoyl chloride (7.4g, Aldrich) in DCM (35ml) was added dropwise at 0 °C over 30 minutes to a stirred mixture of 30% aqu. methylamine (4.5ml) and triethylamine (3.6g) in DCM (50ml). When addition was complete, the mixture was allowed to warm to room temperature over 1 hour with stirring. Further methylamine (0.5ml) was added and the mixture stirred for a further 10 minutes, then washed with water, 1N aqu. HCl, 1N aqu. NaOH and brine, dried over $MgSO_4$ and evaporated in vacuo to give the desired product as a colourless oil which solidified to a white solid on standing.

(c) N-Methyl-3-[4-(4-nitrobenzyl)-2,5-dioxoimidazolidinylmethyl]benzamide

The product from step (a) (4.0g), the product from step (b) (3.3g) and anhy. $K_2CO_3$ (2.55g) were taken up in DMF (25ml) and stirred at room temperature for 60 hours. The mixture was poured with vigorous stirring into ice-water (175ml) to give an oily precipitate. Ethyl acetate was added and stirring continued for 1 hour to solidify the precipitate. The latter was filtered off, washed with water, ethyl acetate and ether, and dried to give the desired product as a peach-coloured solid (4.5g).

Synthetic Example 2

Preparation of (-)-4′-{2-[4-(4-Nitrobenzyl)-2,5-dioxoimidazolidiny]ethyl}acetanilide

(a) (-)-4-(4-Nitrobenzyl)imidazolidin-2,5-dione

By the method of Synthetic Example 1, step (a), using p-nitro-L-phenylalanine (Fluka). $[\alpha]_D^{25}$ -88.2° (c = 0.50, MeOH).

(b) 2-(4-Acetamidophenyl)ethyl acetate

A solution of acetyl chloride (35.6ml) in dioxan (110ml) was added dropwise at 0 °C to a stirred mixture of p-aminophenethyl alcohol (34.3g, Aldrich) and triethylamine (37.5g) in dioxan (110ml). When addition was complete, the mixture was stirred at room temperature for 17 hours, then poured into 2N aqu. HCl, saturated with NaCl, and extracted with ethyl acetate (x3). The combined extracts were washed with water and brine, dried over $MgSO_4$ and evaporated in vacuo to give the desired product as a brown solid (55.3g).

(c) 4′-(2-Hydroxyethyl)acetanilide

A solution of the product from step (b) (55.3g) in methanol (250ml) and 1N aqu. NaOH (500ml) was stirred at room temperature for 1 hour. The methanol was evaporated in vacuo and the remaining aqueous solution adjusted to pH 4 (2N aqu. HCl), saturated with NaCl, and extracted with ethyl acetate (x3). The combined extracts were washed with water and brine, dried over $MgSO_4$ and evaporated in vacuo to give a brown oil which was flash chromatographed through a silica column using ethyl acetate and then crystallised from ethyl acetate to give the desired product (51.7g).

(d) (-)-4′-{2-[4-(4-Nitrobenzyl)-2,5-dioxoimidazolidinyl]ethyl}acetanilide

A solution of diethylazodicarboxylate (7.8g) in DME (40ml) was added dropwise under $N_2$ to a stirred mixture of the product from step (a) (10.5g), the product from step (c) (8.0g) and triphenylphosphine (11.7g) in DME (400ml). When addition was complete, the mixture was stirred at room temperature for 17 hours, then evaporated in vacuo and the residue crystallised from ethanol/ether (1:4 v/v) to give the desired product as a pale yellow solid (12.7g), $[\alpha]_D^{25}$ -97.8° (c = 0.50, MeOH).

Synthetic Example 3

Preparation of 1-Benzyl-3-(4-nitrobenzyl)imidazolidin-2,4-dione

(a) 3-(4-Nitrobenzyl)imidazolidin-2,4-dione
A solution of hydantoin (5.0g, Aldrich) in 2N aqu. NaOH (25ml) was stirred at room temperature for 1 hour, then a solution of p-nitrobenzyl bromide (10.8g, Aldrich) in methanol (50ml) was added and the mixture refluxed for 16 hours. The mixture was cooled and the pale yellow precipitate filtered off, dried in vacuo, and recrystalised from ethanol to give the desired product as pale yellow crystals (6.8g).
(b) 1-Benzyl-3-(4-nitrobenzyl)imidazolidin-2,4-dione
A solution of the product from step (a) (5.9g) in DMF (15ml) was added under $N_2$ to a stirred suspension of anhy. $K_2CO_3$ (3.5g) in DMF (15ml), followed by the addition of benzyl bromide (4.3g, Aldrich). After additions were complete, the mixture was stirred at room temperature for 16 hours, then poured into ice-water and extracted with ethyl acetate (x3). The combined extracts were washed with brine, dried over $MgSO_4$ and evaporated to give a yellow oil which was flash chromatographed through a silica column using ethyl acetate to give the desired product as a yellow oil (7.0g).

Synthetic Example 4

Preparation of 1-Benzyl-3-(4-nitrobenzyl)pyrrolidin-2,5-dione

A solution of benzylamine (1.2g, Aldrich) in DCM (10ml) was added dropwise over 5 minutes to a stirred suspension of p-nitrobenzylsuccinic anhydride (2.4g, JCS Perkin I, 1975, 830) in DCM (10ml). After initial exothermicity, mixture stirred at room temperature for 3 hours, then evaporated in vacuo to give a colourless foam to which benzene (20ml) and acetyl chloride (5ml) were added. The mixture was stirred for 20 minutes at room temperature, then refluxed for 5 hours. The acetyl chloride was distilled off to leave 10ml of reaction mixture from which crystalline solid deposited on cooling. The solid was filtered off and washed with benzene to give the desired product as a very pale yellow solid (2.1g), mp 135-137°C. The 200MHz $^1H$ NMR was consistent with the proposed structure.

Synthetic Example 5

Preparation of 3-Benzyl-5-(4-nitrobenzyl)oxazolidin-2,4-dione

(a) 2-Hydroxy-3-(4-nitrophenyl)propionic acid
A solution of sodium nitrite (4.6g) in water (18ml) was added dropwise at 0°C over 20 minutes to a stirred mixture of p-nitrophenylalanine (6.3g, Fluka) c.HCl (3.1ml), 5% aqu. $H_2SO_4$ (80ml) and water (10ml). When addition was complete, the mixture was stirred at 0°C for 1 hour, then allowed to warm to room temperature over 2 hours. The mixture was extracted with ethyl acetate (x4) and the extracts dried over $MgSO_4$ and evaporated in vacuo to give the desired product as a white solid (3.1g).
(b) Methyl 2-hydroxy-3-(4-nitrophenyl)propionate
A solution of the product from step (a) (3.0g) and c.HCl (0.5ml) in methanol (100ml) was refluxed for 1 hour. The solvents were evaporated in vacuo and the residue flash chromatographed through a silica column using methanol/chloroform (3:100 v/v) to give the desired product as a pale yellow solid (2.7g). The 200MHz $^1H$ NMR was consistent with the proposed structure.
(c) 3-Benzyl-5-(4-nitrobenzyl)oxazolidin-2,4-dione
Benzyl isocyanate (1.2g, Aldrich) was added at room temperature to a stirred solution of the product from step (b) (2.03g) in toluene (25ml). When addition was complete, the mixture was refluxed for 40 hours. The toluene was evaporated in vacuo and the residue flash chromatographed through a silica column using chloroform to give the desired prodcut as a colourless oil which crystallised on standing (2.9g). The product was recrystallised from IPA as fine white needles (1.8g), mp 92-93°C. The 200MHz $^1H$ NMR was consistent with the proposed structure.

Synthetic Example 6

Preparation of 1-Benzyl-4-(4-nitrobenzyl)imidazolidin-2,5-dione

Benzyl isocyanate (3.2g, Aldrich) was added dropwise at 0 °C over 20 minutes to a stirred mixture of p-nitrophenylalanine (4.2g Fluka) and KOH (1.3g) in water (40ml). When addition was complete, the mixture was stirred at 60-70 °C for 2 hours, then cooled, filtered, and the filtrate treated with c.HCl. The resulting white solid was filtered off, washed with ice-water, dried in vacuo, then suspended in 50% aqu. HCl (20ml) and refluxed for 2 hours. The suspension was cooled, diluted with water, filtered, and the residue dried in vacuo to give the desired product (5.7g).

Synthetic Example 7

Preparation of 5-Benzyl-3-[2-(4-nitrophenyl)ethyl]imidazolidin-2,4-dione

(a) 5-Benzylimidazolidin-2,4-dione
A solution of DL-β-phenylalanine (4.9g, Aldrich) and potassium cyanate (2.4g) in water (9ml) was refluxed for 1 hour. C.HCl (4.5ml) was added and the mixture refluxed for a further 10 minutes, then cooled, diluted with water, and the resulting precipitate filtered off, washed with ice-water, and dried in vacuo to give the desired product as a crystalline white solid (4.0g).
(b) 5-Benzyl-3-[2-(4-nitrophenyl)ethyl]imidazolidin-2,4-dione
A solution of the product from step (a) (4.0g) in DMF (10ml) was added under $N_2$ to a stirred suspension of anhy. $K_2CO_3$ (3.0g) in DMF (10ml), followed by the addition of a solution of p-nitrophenethyl bromide (4.9g, Aldrich) in DMF (5ml). After additions were complete, the mixture was stirred at room temperature for 17 hours, then at 40 °C for 48 hours. The mixture was poured into ice-water, stirred for 2 hours, and the resulting precipitate filtered off, washed with water, and triturated with ethanol to give the desired product as a pale yellow solid (3.7g).

Synthetic Example 8

Preparation of 3-Benzyl-1-(4-nitrobenzyl)imidazolidin-2,4-dione

A solution of 3-benzylimidazolidin-2,4-dione (5.7g, J.Org.Chem. 1965, 3414) in DMF (10ml) was added under $N_2$ to a stirred suspension of anhy. $K_2CO_3$ (4.1g) in DMF (10ml), followed by the addition of a solution of p-nitrobenzyl bromide (6.5g, Aldrich) in DMF (10ml). After additions were complete, the mixture was stirred at room temperature for 72 hours, then poured into ice-water and extracted with ethyl acetate (x3). The combined extracts were washed with water (x2) and brine, dried over $MgSO_4$ and evaporated to give a yellow oil which was flash chromatographed through a silica column using chloroform to give the desired product as a pale yellow solid (7.9g).

Synthetic Example 9

Preparation of 4-Benzyl-1-(4-nitrobenzyl)-1,2,4-triazolidin-3,5-dione

A mixture of 4-benzylurazole (3.8g, JCS Perkin II, 1975, 1325), p-nitrobenzyl bromide (4.3g, Aldrich) and polymer-supported 1,5,7-triazabicyclo[4.4.0]dec-5-ene (P-TBD, 6.3g, Fluka) in DCM (400ml) was refluxed under $N_2$ for 24 hours. The P-TBD was filtered off and the filtrate evaporated to leave a gum which was flash chromatographed through a silica column using chloroform/methanol (19:1 v/v) to give the desired product as a solid (3.8g).

Synthetic Example 10

Preparation of 3-Benzyl-1-(4-nitrobenzyl)pyrrolidin-2,5-dione

(a) Triethyl 3-phenylpropane-1,2,2-tricarboxylate
Sodium (2.3g) was dissolved in dry ethanol (140ml), triethylethane-1,1,2-tricarboxylate (24.8g, Aldrich) and benzyl chloride were (12.8g) added, and the mixture was stirred under reflux for 18 hours, then filtered and evaporated in vacuo. Water (100ml) was added to the residue and extracted with ether (x3).

The combined extracts were washed with water, dried over $MgSO_4$ and evaporated in vacuo to give the desired product as a light yellow oil (32.6g).

(b) Diethyl 3-phenylpropane-1,2-dicarboxylate

A solution of the product from step (a) (23,6g) in c.HCl (326ml) was stirred under reflux for 46 hours, then cooled. The resulting precipitate was extracted with chloroform (x3) and the extracts combined, washed with 2M aqu. KOH (x2), acidified to pH 4.0 (2M aqu. HCl) and cooled. The resulting precipitate was filtered off, washed with water and dried in vacuo to give the desired product (15.7g). The 200MHz $^1H$ NMR was consistent with the proposed structure.

(c) 3-Benzylsuccinic anhydride

The product from step (b) was suspended in acetyl chloride (24ml) and the suspension refluxed for 3 hours, then evaporated in vacuo to give a white solid which was triturated and washed with 60-80 petrol and dried in vacuo to give the desired product (9.7g). The 200MHz $^1H$ NMR was consistent with the proposed structure.

(d) 3-Benzyl-1-(4-nitrobenzyl)pyrrolidin-2,5-dione

A suspension of p-nitrobenzylamine hydrochloride (8.1g), Aldrich), and triethylamine (4.3g) in DCM (40ml) was added dropwise to a stirred solution of the product from step (c) (7.6g) in DCM (40ml) and the mixture stirred at room temperature for 3 hours, then evaporated in vacuo to give a foam to which benzene (82ml) and acetyl chloride (20.5ml) were added. The mixture was stirred for 20 minutes at room temperature, then refluxed for 5 hours and evaporated in vacuo. The residue was taken up in DCM (400ml), washed with water (x3), dried over $MgSO_4$ and evaporated in vacuo. The residue was triturated with petrol and dried in vacuo to give the desired product (12.7g). The 200MHz $^1H$ NMR was consistent with the proposed structure.

Synthetic Example 11

Preparation of (-)-5-(4-nitrobenzyl-3-(3-phenoxybenzyl)imidazolidin-2,4-dione

(a) 3-Phenoxybenzamide

Triethylamine (5.6g) was added at 0°C under $N_2$ to a stirred solution of 3-phenoxybenzoic acid (10.7g, Aldrich) in THF (75ml), followed by the addition of methyl chloroformate (4.7g) at less than 5°C. After additions were complete, the mixture was stirred at 0°C for 30 minutes. 0.88 Ammonia (30ml) was added and the mixture stirred at room temperature for 20 hours, then evaporated in vacuo and the residue taken up in ethyl acetate (100ml) and water (100ml). The aqueous phase was separated, extracted with ethyl acetate and the combined ethyl acetate solutions washed with 5% w/v aqu. $Na_2CO_3$ (2x50ml), 2N aqu. HCl (50ml) and water (100ml), dried over $Na_2SO_4$ and evaporated in vacuo to give the desired product as a colourless solid which was recrystallised from ethyl acetate/60-80 pet. ether (1:2 v/v), mp 125-127°C (6.5g). The 200MHz $^1H$ NMR was consistent with the proposed structure.

(b) 3-Phenoxybenzylamine

A solution of the product from step (a) (6.5g) in THF (75ml) was added under $N_2$ over 15 minutes to a stirred suspension of LAH (2.3g) in THF (100ml). After addition was complete, the mixture was refluxed for 5.5 hours, then cooled to -5°C, excess LAH destroyed by the careful addition of 15% w/v aqu. NaOH (100ml), and the mixture taken up in water (150ml) and ether (200ml). The aqueous phase was separated, extracted with ether and the combined ether solutions washed with 2N aqu. NaOH (2x100ml) and water (2x200ml), dried over $Na_2SO_4$ and evaporated in vacuo to give the desired product as a colourless oil (5.9g). The 200MHz $^1H$ NMR was consistent with the proposed structure.

(c) (+)-Methyl 2-amino-3-(4-nitrophenyl)propionate hydrochloride

A solution of p-nitro-L-phenylalanine (12.6g) and c.HCl (7.5g) in methanol (150ml) was refluxed for 1.5 hours and the solvent then evaporated in vacuo. The residue was triturated with ether and recrystallised from SVM to give the desired product as a off-white solid, $[\alpha]^{25}$ +15.3° (c = 0.55, MeOH).

(d) (+)-Methyl 2-isocyanato-3-(4-nitrophenyl)propionate

Phosgene was bubbled slowly into a refluxing suspension of the product from step (c) (5.0g) in toluene (75ml) for 6 hours. The mixture was cooled, filtered and the residue washed with toluene. The combined filtrate and washings were evaporated in vacuo to give the desired product as a white solid (2.0g).

(e) (+)-1-[1-Methoxycarbonyl-2-(4-nitrophenyl)ethyl]-3-(3-phenoxybenzyl)urea

A solution of the product from step (b) (1.6g) in DCM (1ml) was added at 0°C under $N_2$ to a stirred solution of the product from step (d) (2.0g) in DCM (20ml). After addition was complete, the mixture was allowed to warm to room temperature over 1 hour with stirring. The mixture was evaporated in vacuo and the residue flash chromatographed through a silica column using methanol/chloroform (2:100 v/v) to give

the desired product as a pale yellow solid which was recrystallised from DCM/ether (1:2 v/v). Mp 112-114°C, $[\alpha]_D^{25}$ +2.6° (c = 0.51, MeOH), elemental analysis in accordance with proposed structure.

(f) (-)-5-(4-Nitrobenzyl-3-(3-phenoxybenzyl)imidazolidin-2,4-dione

A suspension of the product from step (e) (2.0g) in 5N aqu. HCl (70ml) was heated at 100°C for 1.5 hours. The resulting off-white precipitate was filtered off, washed with water, dried and recrystallised from ethanol to give the desired product as white needles (1.3g), $[\alpha]_D^{25}$ -16.0° (c = 0.51, MeOH).

Synthetic Example 12

Compounds of formula (V) wherein Y is >NH may be converted to compounds of formula (V) wherein Y is >$NR^5$, where $R^5$ is as hereinbefore defined other than hydrogen.

By way of example, the compound of formula (V) obtained in Synthetic Example 6 may be N-methylated in the hydantoin ring by the following method.

Preparation of 1-Benzyl-3-methyl-4-(4-nitrobenzyl)imidazolidin-2,5-dione

Methyl iodide (3.5g) was added dropwise under $N_2$ to a stirred mixture of the compound of formula (V) obtained in Synthetic Example 6 and anhy. $K_2CO_3$ (1.9g) in DMF (20ml). After addition was complete, the mixture was stirred at room temperature for 24 hours. Further methyl iodide (0.8ml) was added and the mixture stirred for a further 24 hours, then poured into water and extracted with ethyl acetate (x3). The combined extracts were washed with water (x2) and brine, dried over $MgSO_4$ and evaporated to give a yellow oil which was flash chromatographed through a silica column using chloroform. The desired product was obtained as a white crystalline solid (3.0g).

Synthetic Examples 13

Compounds of formula (V) wherein Z and/or Z′ is >C=O may be converted to compounds of formula (IV) wherein Z and/or Z′ is >$CH_2$.

By way of example, the compounds of formula (V) obtained in Synthetic Examples 8 and 12 may be converted from imidazolidin-2,4-diones to imidazolidin-2-ones by the following methods.

Preparation of 1-(4-Aminobenzyl)-3-benzylimidazolidin-2-one hydrochloride

(a) 1-Benzyl-3-(4-nitrobenzyl)-4-imidazolin-2-one

A suspension of LAH (370mg) in THF (35ml) was added under $N_2$ over 1 hour to a stirred solution of the compound of formula (V) obtained in Synthetic Example 8 (3.2g) in THF (35ml). After addition was complete, the mixture was stirred at room temperature for 17 hours, then 2N aqu. HCl (6ml) was added, followed by c.HCl (2ml). The resulting organic phase was separated and washed with brine, dried over $MgSO_4$ and evaporated in vacuo to give the desired product as a yellow oil (3.0g).

(b) 1-(4-Aminobenzyl)-3-benzylimidazolidin-2-one hydrochloride

A mixture of the product from step (a) (3.0g), 10% Pd/C (750mg) and 2N aqu. HCl (4.7ml) in ethanol/water (55ml, 3:2 v/v) was hydrogenated at room temperature and atmospheric pressure for 24 hours (uptake 1100 ml). The mixture was filtered through celite, the residue washed with hot water and the filtrate evaporated in vacuo to give the desired product as a yellow oil (2.9g). The 200MHz $^1H$ NMR and MS were consistent with the proposed structure.

Preparation of 4-(4-Aminobenzyl)-1-benzyl-3-methylimidazolidin-2-one hydrochloride

(a) 1-Benzyl-3-methyl-4-(4-nitrobenzyl)imidazolin-2-one

A solution of glac. acetic acid (2.0ml) in IPA (8.3ml) was added dropwise over 25 minutes to a stirred suspension of the compound of formula (V) obtained in Synthetic Example 12 (2.3g) and sodium borohydride (1.3g) in IPA (22ml). After addition was complete, the mixture was stirred at room temperature for 20 hours. More IPA (30ml) was added and the mixture refluxed for 4 hours, then cooled and more sodium borohydride (1.3g) added. A further solution of glac. acetic acid (2.0ml) in IPA (8.3ml) was added dropwise to the stirred mixture. After addition was complete, the mixture was refluxed for 4 hours, then poured into 2N aqu. HCl and the latter extracted with ether (x3). The combined extracts were washed with brine, dried over $MgSO_4$ and evaporated in vacuo to give the desired product as a yellow oil (2.2g).

(b) 4-(4-Aminobenzyl)-1-benzyl-3-methylimidazolidin-2-one hydrochloride

A mixture of the product from step (a) (2.2g), 10% Pd/C (400mg) and 2N aqu. HCl (4.3ml) in ethanol/water (27.8ml, 3:5 v/v) was hydrogenated at room temperature and atmospheric pressure for 24 hours (uptake ca 200ml). The mixture was filtered through celite, the residue washed with hot water and the filtrate evaporated in vacuo to give the desired product as a white solid (2.1g).

Synthetic Example 14

The compounds of formula (V) obtained in Synthetic Examples 1 to 12 may be converted to compounds of formula (I) by (i) reduction to the corresponding aniline, (ii) conversion of the aniline to the corresponding hydrazine, and (iii) cyclisation of the hydrazine to a compound of formula (I) by the Fischer indole synthesis. The compounds of formula (IV) obtained in Synthetic Example 13 require only steps (ii) and (iii).

By way of example, the compounds of formula (V) obtained in Synthetic Examples 1 and 4 may be converted to compounds of formula (I) by the following methods.

Preparation of (±)-2-{5-[1-(3-Methylaminocarbonylbenzyl)-2,5-dioxoimidazolidin-4-ylmethyl]-1H-indol-3-yl}ethylamine

(a) 3-[4-(4-Aminobenzyl)-2,5-dioxoimidazolidinylmethyl]-N-methylbenzamide hydrochloride

A mixture of the product from step (c) in Synthetic Example 1 (4.5g), 10% Pd/C (250mg) and 2N aqu. HCl (5.7ml) in ethanol/water (75ml, 3:2 v/v) was hydrogenated at room temperature and atmospheric pressure for 5 hours (uptake 1200ml). The mixture was filtered through Hyflo, the residue washed with water and the filtrate evaporated in vacuo to give the desired product as a pale mustard-coloured solid (4.6g).

(b) 3-[4-(4-Hydrazinobenzyl)-2,5-dioxomidazolidinylmethyl]-N-methylbenzamide hydrochloride

A solution of sodium nitrite (0.80g) in water (8ml) was added dropwise at -10 °C over 15 minutes to a stirred suspension of the product from step (a) (4.5g) in c.HCl (28ml) and water (16ml). After addition was complete, the mixture was stirred at 0 °C for 15 minutes, then added dropwise at -10 °C over 15 minutes to a stirred solution of tin(II) chloride dihydrate in c.HCl (23ml). After addition was complete, the mixture was allowed to warm to room temperature over 1 hour. The resulting precipitate was filtered off at 0 °C, washed with cold ether, and dried in vacuo to give the desired product as a white solid (4.6g).

(c) (±)-2-{5-[1-(3-Methylaminocarbonylbenzyl)-2,5-dioxoimidazolidin-4-yl methyl]-1H-indol-3-yl}ethylamine

A mixture of the product from step (b) (1.21g) and 4-chlorobutanal dimethyl acetal (456mg, JACS 1951, 1365) in ethanol/water (60ml, 5:1 v/v) was refluxed for 2 hours, then evaporated in vacuo and the residue flash chromatographed through a silica column using DCM/ethanol/ammonia (20:8:1 v/v/v) to give the desired product as a pale cream coloured oil (275mg).

Preparation of (±)-2-[5-(1-Benzyl-2,5-dioxopyrrolidin-3-ylmethyl)-1H-indol-3-yl]ethylamine

(a)′ 3-(4-Aminobenzyl)-1-benzylpyrrolidin-2,5-dione hydrochloride

A mixture of the product from step (a) of Synthetic Example 4 (5.0g), tin(II) chloride dihydrate (17.4g) and c.HCl (3ml) in ethanol (100ml) was refluxed under $N_2$ for 3.5 hours, then poured into satd. aqu. $NaHCO_3$ (400ml) and the mixture saturated with NaCl, basified to pH 8 by the addition of more satd. aqu. $NaHCO_3$, and extracted with ethyl acetate (x3). The combined extracts were washed with water (x2) and brine (x2), dried over $MgSO_4$ and evaporated in vacuo. The residue was crystallised from ethyl acetate/60-80 petrol (1:2 v/v) and air dried to give the desired product (2.9g).

(b)′ 1-Benzyl-3-(4-hydrazinobenzyl)pyrrolidin-2,5-dione hydrochloride

By analogy with step (b).

(c)′ (±)-2-[5-(1-Benzyl-2,5-dioxopyrrolidin-3-ylmethyl)-1H-indol-3-yl]ethylamine

By analogy with step (c).

Synthetic Examples 15

Compounds of formula (I) wherein $R^3 = R^4 = H$ may be converted to corresponding compounds of formula (I) wherein $R^3$ and/or $R^4$ are as hereinbefore defined other than hydrogen.

By way of example, the compound of formula (I) obtained in Synthetic Example 14, step (c), may be N-alkylated and the compound of formula (I) derived from the compound of formula (V) obtained in Example 2

may be N,N-dialkylated by the following methods.

Preparation of (±)-N-Methyl-2-{5-[1-(3-methylaminocarbonylbenzyl)-2,5-dioxoimidazolidin-4-ylmethyl]-1H-indol-3-yl}ethylamine

(a) N-Benzyl-2-{5-[1-(3-methylcarbamoylbenzyl)-2,5-dioxoimidazolidin-4-yl methyl]-1H-indol-3-yl}ethylamine

A mixture of the compound of formula (I) obtained in step (c) of Synthetic Example 14 (860mg) and benzaldehyde (224mg) in ethanol (9ml) was stirred at room temperature for 30 hours. Sodium borohydride (80mg) was added portionwise over 10 minutes and the mixture then stirred at room temperature for 1 hour. The solvent was evaporated in vacuo and the residue taken up in 2N aqu. HCl, basified with $NaHCO_3$, saturated with $K_2CO_3$, and extracted with ethyl acetate (x3). The combined extracts were dried over $MgSO_4$ and evaporated in vacuo to give a pale yellow foam which was flash chromatographed through a silica column using DCM/ethanol/ammonia (50:8:1 v/v/v) to give the desired product as a colourless oil (623mg).

(b) N-Benzyl-N-methyl-2-{5-[1-(3-methylcarbamoylbenzyl)-2,5-dioxoimidazolidin-4-ylmethyl]-1H-indol-3-yl}ethylamine

Dimethyl sulphate (168mg) was added dropwise under $N_2$ to a stirred mixture of the product from step (a) (646mg) and anhy. $K_3CO_3$ (424mg) in DMF (25ml). After addition was complete, the mixture was stirred for 4 hours at room temperature, then poured into water and extracted with ethyl acetate (x7). The combined extracts were washed with water and brine, dried over $MgSO_4$ and evaporated in vacuo to give a yellow oil which was flash chromatographed through a silica column using DCM/ethanol/ammonia (50:8:1 v/v/v) to give the desired product (333mg).

(c) (±)-N-Methyl-2-{5-[1-(3-methylaminocarbonylbenzyl)-2,5-dioxoimidazolidin-4-ylmethyl]-1H-indol-3-yl}ethylamine

A mixture of the product from step (b) (257mg) and 10% Pd/C (110mg) in ethanol (16ml) was hydrogenated at room temperature and atmospheric pressure for 6 hours (uptake 12ml). The mixture was filtered through celite, the residue washed with ethanol and the filtrate evaporated in vacuo to give a colourless oil which was flash chromatographed through a silica column using DCM/ethanol/ammonia (50:8:1 v/v/v) to give the desired product as a colourless oil (87mg).

Preparation of (-)-N,N-Dimethyl-2-[5-{1-[2-(4-acetylaminophenyl)ethyl]-2,5-dioxoimidazolidin-4-ylmethyl}-1H-indol-3-yl]ethylamine

A solution of formaldehyde (24mg) in methanol (3.6ml) was added dropwise under $N_2$ to a stirred mixture of the compound of formula (I) derived from the compound of formula (V) obtained in Synthetic Example 2 (171mg), sodium cyanoborohydride (30mg) and glac. acetic acid (120mg) in methanol (3.6ml). After addition was complete, the mixture was stirred for 2.5 hours, then satd. aqu. $K_2CO_3$ was added and the mixture extracted with ethyl acetate (x3). The combined extracts were washed with brine, dried over $MgSO_4$ and evaporated to give a white foam which was flash chromatographed through a silica column using DCM/ethanol/ammonia (30:8:1 v/v/v) to give the desired product as a colourless oil (155mg).

Synthetic Example 16

Compounds of formula (I) may be converted to corresponding salts by treatment with the appropriate acids.

By way of example, the compound of formula (I) derived from the compound of formula (V) obtained in Synthetic Example 4 may be converted to the maleate salt by the following method.

Preparation of (±)-2-[5-(1-Benzyl-2,5-dioxopyrrolidin-3-ylmethyl)-1H-indol-3-yl]ethylamine maleate dihydrate

A solution of maleic acid (19mg) and the compound of formula (I) derived from the compoun of formula (V) obtained in Synthetic Example 4 (58.5mg) in methanol (3ml) was stood for 2.5 hours, evaporated in vacuo and the resulting solid freeze-dried from water to give the desired product (44mg).

Synthetic Examples 17 to 28

By steps analogous to those described in Synthetic Examples 13 to 16, the compounds of formula (V) obtained in Synthetic Examples 1 to 12 were converted to the following compounds of formula (I). The 200MHz $^1$H NMR, MS and elemental analysis for each compound were consistent with the proposed structure.

17) (±)-2-{5-[1-(3-Methylaminocarbonylbenzyl)-2,5-dioxoimidazolidin-4-ylmethyl)-1H-indol-3-yl}ethylamine. 1.5 maleate. 0.5 ethyl acetate, mp 89-94°C;

18) (-)-N,N-Dimethyl-2-[5-{1-[2-(4-acetylaminophenyl)ethyl]-2,5-dioxoimidazolidin-4-ylmethyl}-1H-indol-3-yl]ethylamine maleate hydrate, mp 71°C (softens 56°), $[\alpha]_D^{25}$ -18.3° (c = 0.51, MeOH);

19) (±)-2-[5-(1-Benzyl-2,5-dioxopyrrolidin-3-ylmethyl)-1H-indol-3-yl] ethylamine maleate dihydrate;

20) (±)-2-{5-[(3-Benzyl-2,4-dioxooxazolidin-5-yl)methyl]-1H-indol-3-yl} ethylamine maleate hydrate;

21) (±)-2-[5-(1-Benzyl-3-methyl-2-oxoimidazolidin-4-ylmethyl)-1H-indol-3-yl]ethylamine. 1.2 maleate. 0.8 hydrate;

22) (±)-2-[5-(5-Benzyl-2,4-dioxomidazolidin-3-ylmethyl)-1H-indol-3-yl]ethylamine maleate hydrate, mp 88-90°C;

23) 2-[5-(3-Benzyl-2,4-dioxoimidazolidin-1-ylmethyl)-1H-indol-3-yl]ethylamine maleate hydrate. 0.5 ethanolate;

24) 2-[5-(4-Benzyl-3,5-dioxo-1,2,4-triazol-2-ylmethyl)-1H-indol-3-yl] ethylamine maleate hydrate;

25) (±)-2-[5-(3-Benzyl-2,5-dioxopyrrolidinylmethyl)-1H-indol-3-yl] ethylamine. 1.1 maleate. 1.3 hydrate;

26) (-)-2-[5-{[2,5-Dioxo-1-(3-phenoxybenzyl)imidazolidin-4-yl]methyl} 1H-indol-3-yl]ethylamine maleate hydrate, $[\alpha]_D^{25}$ -11.4° (c = 0.52, MeOH);

27) (±)-N-Methyl-2-{5-[1-(3-methylaminocarbonylbenzyl)-2,5-dioxoimidazolidin-4-ylmethyl]-1H-indol-3-yl}ethylamine maleate hydrate;

28) (-)-N,N-Dimethyl-2-{5-[2,5-dioxo-1-(3-phenoxybenzyl)imidazolidin-4-yl methyl]-1H-indol-3-ylethylamine maleate. 1.5 hydrate, $[\alpha]_D^{25}$ -7.2° (c = 0.56, MeOH);

Synthetic Examples 29 to 89

By steps analogous to those described in Synthetic Examples 1 to 16, the following compounds of formula (I) were prepared. The 200MHz $^1$H NMR, MS and elemental analysis for each compound were consistent with the proposed structure.

29) (±)-2-[5-(1-Benzyl-2,5-dioxoimidazolidin-4-yl)-1H-indol-3-yl]ethylamine hydrochloride, mp 233-240°C (dec. 173-174°C);

30) (±)-2-{5-[2,4-Dioxo-3-(2-phenylethyl)imidazolidin-5-ylmethyl]-1H-indol-3-yl}ethylamine maleate. 0.5 hydrate, mp 98-100°C;

31) (±)-2-{5-[1-(4-Biphenylylmethyl)-2,5-dioxoimidazolidin-4-ylmethyl]-1H-indol-3-yl}ethylamine maleate. 1.5 hydrate, mp 132-134°C (softens 115°C);

32) (±)-2-[5-(4-Benzyl-2,5-dioxoimidazolidin-1-yl)-1H-indol-3-yl]ethylamine maleate hydrate, mp 130-135°C;

33) (±)-N,N-Dimethyl-2-[5-(1-phenylethyl-2,5-dioxoimidazolidin-4-ylmethyl)-1H-indol-3-yl]ethylamine maleate. 1.5 hydrate, mp 65-70°C;

34) (±)-2-[5-(3-Thenyl-2,4-dioxoimidazolidin-5-ylmethyl)-1H-indol-3-yl]ethylamine maleate;

35) (±)-3-[3-(2-Aminoethyl)-1H-indol-5-ylmethyl]-5-benzylimidazolidin-2,4-dione maleate. 1.3 hydrate, mp 111-113°C (softens 86°C);

36) (±)-2-[5-{[2,5-Dioxo-1-(3-phenoxybenzyl)imidazolidin-4-yl]methyl}-1H-indol-3-yl]ethylamine maleate dihydrate, softens 75°C;

37) (±)-2-{5-[(1,3-Dibenzyl-2,5-dioxoimidazolidin-4-yl)methyl]-1H-indol-3-yl}ethylamine. 1.5 maleate. 1.5 hydrate;

38) (±)-2-[5-(3-Benzyl-1-methyl-2,5-dioxoimidazolidin-4-ylmethyl)-1H-indol-3-yl]ethylamine maleate. 0.5 hydrate;

39) (±)-2-{5-[2,5-Dioxo-1-(3-phenylpropyl)imidazolidin-4-ylmethyl]-1H-indol-3-yl}ethylamine maleate. 0.8 hydrate;

40) (±)-2-{5-[2-(1-Benzyl-2,5-dioxoimidazolidin-4-yl)ethyl]-1H-indol-3-yl}ethylamine maleate. 0.8 hydrate, mp 139°C;

41) (±)-N,N-Dimethyl-2-{5-[2,5-dioxo-1-(3-phenoxybenzyl)imidazolidin-4-ylmethyl]-1H-indol-3-ylethylamine maleate hydrate;

42) (±)-2-[5-(1-Benzyl-3-methyl-2,5-dioxoimidazolidin-4-ylmethyl)-1H-indol-3-yl]ethylamine maleate hydrate;

43) (±)-2-[5-{[2,5-Dioxo-1-(4-phenylsulphonybenzyl)imidazolidin-4-yl]methyl}-1H-indol-3-yl]ethylamine maleate. 1.3 hydrate, softens 80°C (dec. 153-155°C);

44) (±)-2-[5-{[2,5-Dioxo-1-(3-phenylsulphonybenzyl)imidazolidin-4-yl]methyl}-1H-indol-3-yl]ethylamine maleate. 1.3 hydrate, mp 137-139°C (softens 118°C);

45) (±)-2-[5-{2,5-Dioxo-1-[3-(9-oxafluorene)methyl]imidazolidin-4-yl}methyl-1H-indol-3-yl]ethylamine maleate. 1.3 hydrate, dec. 124°C;

46) (±)-N-{4-[5-(3-(2-Aminoethyl)-1H-indol-5-yl)-2,5-dioxoimidazolidin-4-ylmethyl]methyl}phenylacetamide maleate hydrate, dec. 163-165°C;

47) (±)-2-[5-{(1-(3-Benzamidobenzyl)-2,5-dioxoimidazolidin-4-yl]methyl}-1H-indol-3-yl]ethylamine maleate dihydrate, softens 142°C (dec. 175-177°C);

48) (±)-2-[5-{[1-(4-Anilinocarbonylbenzyl)-2,5-dioxoimidazolidin-4-yl]methyl}-1H-indol-3-yl]ethylamine maleate. 1.8 hydrate, softens 122-124°C (dec. 168-172°C);

49) (±)-2-[5-{2-[2,5-Dioxo-1-(thien-2-ylmethyl)imidazolidin-4-yl]ethyl}-1H-indol-3-yl]ethylamine. 1.2 maleate hydrate, mp 145-146°C (softens 140°C);

50) (±)-2-{5-[1-(4-Methoxybenzyl)-2,5-dioxoimidazolidin-4-ylmethyl]-1H-indol-3-yl}ethylamine maleate. 1.3 hydrate, mp 149-151°C (softens 100°C);

51) (±)-N,N-Dimethyl-2-{5-[2-(2,5-Dioxo-1-thienylmethylimidazolidin-4-yl)ethyl]-1H-indol-3-yl}ethylamine maleate hydrate;

52) (±)-N,N-Di-n-propyl-2-{5-[2-(2,5-Dioxo-1-thienylmethylimidazolidin-4-yl)ethyl]-1H-indol-3-yl}ethylamine maleate hydrate, softens 60°C (dec. 81-83°C);

53) (±)-2-{5-[1-(4-Chlorobenzyl)-2,5-dioxoimidazolidin-4-ylmethyl]-1H-indol-3-yl}ethylamine maleate hydrate, softens 104°C (dec. 130°C);

54) (±)-2-{4-[3-(2-Aminoethyl)-1H-indol-5-ylmethyl]-2,5-dioxoimidazolidin-1-ylmethyl}-N-methylbenzamide maleate dihydrate;

55) (±)-3-[3-(2-Aminoethyl)-1H-indol-5-ylmethyl]-5-thenylimidazolidin-2,4-dione maleate, dec. 194-196°C;

56) (±)-N-{3-[4-{[3-(2-Dimethylaminoethyl)-1H-indol-5-yl]methyl}-2,5-dioxoimidazolidin-1-ylmethyl]-phenyl}benzamide maleate ethanolate hydrate, dec. 116-122°C;

57) (±)-N,N-Dimethyl-2-[5-{[2,5-dioxo-1-(thien-2-ylmethyl)imidazolidin-4-yl]methyl}-1H-indol-3-yl]-ethylamine maleate, mp 114-116°C (softens 80°C);

58) (±)-2-[5-{[2,5-Dioxo-1-(3-phenylaminocarboxybenzyl)imidazolidin-4-yl]-methyl}-1H-indol-3-ylethylamine. 1.3 maleate. 1.3 hydrate, softens 102°C (dec. 162°C);

59) (±)-N,N-Dimethyl-2-{5-[2-(1-benzyl-2,5-dioxoimidazolidin-4-yl)ethyl]-1H-indol-3-yl}ethylamine maleate. 0.5 hydrate, mp 79-82°C;

60) (±)-N,N-Dimethyl-2-{5-[1-(3-methylcarbonylaminobenzyl)-2,5-dioxoimidazolidin-4-ylmethyl]-1H-indol-3-ylmethyl}ethylamine maleate. 1.5 hydrate, mp 128-130°C;

61) (±)-2-{5-[1-(3-Acetylaminobenzyl)-2,5-dioxoimidazolidin-4-ylmethyl]-1H-indol-3-yl)ethylamine. 1.5 maleate hydrate, softens 96°C (dec. 135°C);

62) (±)-2-{5-[1-(2-Acetylaminobenzyl)-2,5-dioxoimidazolidin-4-ylmethyl]-1H-indol-3-yl}ethylamine. 1.5 maleate hydrate, softens 75°C;

63) (±)-2-{5-[1-(3-Isopropylaminocarbonylbenzyl)-2,5-dioxoimidazolin-4-ylmethyl]-1H-indol-3-yl}ethylamine maleate hydrate, mp 120-125°C (dec. 146-150°C);

64) (±)-2-{5-[3-Methyl-2,5-dioxo-4-(2-thenyl)imidazolidin-1-ylmethyl]-1H-indol-3-yl}ethylamine maleate. 0.8 hydrate, softens 90-95°C (dec. 180-182°C);

65) (±)-2-[5-{[2,5-Dioxo-1-(xanthen-3-yl)imidazolidin-4-yl]methyl}-1H-indol-3-yl]ethylamine maleate hydrate, dec. 147°C;

66) (±)-N,N-Dimethyl-2-{5-[1-(3-methylaminocarboxybenzyl)-2,5-dioxoimidazolidin-4-ylmethyl]-1H-indol-3-yl)ethylamine maleate hydrate;

67) (±)-N,N-Dimethyl-2-{5-[3-(3-anilinocarbonylbenzyl)-2,4-dioxoimidazolidin-5-ylmethyl]-1H-indol-3-yl}ethylamine maleate dihydrate, mp 120-125°C;

68) (±)-2-[5-{1-[3-(4-Chlorophenylcarbamoyl)benzyl]-2,5-dioxoimidazolidin-4-ylmethyl)-1H-indol-3-yl]-ethylamine maleate dihydrate, mp 144°C;

69) (±)-2-{5-[1-(3-Methylsulphonylaminobenzyl)-2,5-dioxoimidazolidin-4-ylmethyl]-1H-indol-3-yl}ethylamine maleate. 0.8 hydrate, mp 122°C (softens 104°C);

70) (±)-2-{5-[2,5-Dioxo-1-(2-thien-2-ylethyl)imidazolidin-4-ylmethyl]-1H-indol-3-yl}ethylamine maleate. 1.5 hydrate, dec. 62°C;

71) (±)-2-[5-{1-[2-(4-Methoxyphenyl)ethyl]-2,5-dioxoimidazolidin-4-ylmethyl}-1H-indol-3-yl]ethylamine maleate;

72) (±)-2-[5-{1-[3-(4-Methoxyphenylaminocarbonyl)benzyl-2,5-dioxoimidazolidin-4-ylmethyl)-1H-indol-3-yl]ethylamine maleate. 1.5 hydrate, softens 111 °C (dec. 135-136 °C);

73) (±)-2-[5-{2-[2-(4-Acetylaminophenyl)ethyl]-2,5-dioxoimidazolidin-4-ylmethyl}-1H-indol-3-yl]ethylamine maleate hydrate, mp 126-127 °C;

74) (±)-N,N-Dimethyl-2-{5-[2-(5-Benzyl-2,4-dioxoimidazolidin-3-yl)ethyl]-1H-indol-3-yl}ethylamine maleate. 0.3 hydrate, mp 70-71 °C;

75) (±)-2-{5-[1-(2-N,N-Dimethylcarbamoylbenzyl)-2,5-dioxoimidazolidin-4-ylmethyl]-1H-indol-3-yl}ethylamine maleate. 1.3 hydrate, mp 119-121 °C (softens 93 °C);

76) (±)-2-[5-{2-[1-(3-Acetamidobenzyl)-2,5-dioxoimidazolidin-4-yl]ethyl-1H indol-3-yl]ethylamine. 1.4 maleate. 0.8 hydrate;

77) (±)-N,N-Dimethyl-2-[5-{2-[1-(3-acetamidobenzyl)-2,5-dioxoimidazolidin-4-yl]ethyl}-1H-indol-3-yl]-ethylamine. 1.6 maleate. 1.8 hydrate;

78) (±)-2-[5-{[3-(N-Ethylcarbamoyl)benzyl]-2,5-dioxoimidazolidin-4-ylmethyl}-1H-indol-3-yl]ethylamine. 1.5 maleate hydrate, mp 55 °C (softens 35 °C);

79) (±)-N-Benzyl-N-methyl-2-{5-[1-(3-methylaminocarbonylbenzyl)-2,5-dioxoimidazolidin-4-ylmethyl]-1H-indol-3-yl}ethylamine maleate dihydrate;

80) (±)-2-[5-{2-[1-(4-Acetamidobenzyl)-2,5-dioxomidazolidin-4-yl)ethyl)-H-indol-3-yl]ethylamine. 1.4 maleate. 1.3 hydrate;

81) (±)-2-{5-[1-(4-Cyanobenzyl)-2,5-dioxoimidazolidin-4-ylmethyl]-1H-indol-3-yl}ethylamine maleate hydrate;

82) (±)-N,N-Dimethyl-2-{5-[1-(4-cyanobenzyl)-2,5-dioxoimidazolidin-4-ylmethyl]-1H-indol-3-yl}ethylamine maleate. 1.5 hydrate;

83) (±)-N,N-Diethyl-2-[5-{1-[2-(4-acetamidophenyl)ethyl]-2,5-dioxoimidazolidin-4-ylmethyl}-1H-indol-3-yl]-ethylamine maleate hydrate;

84) (±)-N-Benzyl-N-methyl-2-[5-{1-[2-acetamidophenyl)ethyl]-2,5-dioxoimidazolidin-4-ylmethyl}-1H-indol-3-yl]ethylamine maleate hydrate;

85) (±)-N-Methyl-2-[5-{1-[2-(4-acetamidophenyl)ethyl]-2,5-dioxoimidazolidin-4-ylmethyl}-1H-indol-3-yl]-ethylamine maleate hydrate;

86) (±)-2-{5-[4-(3-Methoxybenzyl)-2,5-dioxoimidazolidinylmethyl]-1H-indol-3-yl}ethylamine maleate hydrate;

87) (±)-2-{5-[4-(4-Methoxybenzyl)-2,5-dioxoimidazolidinylmethyl]-1H-indol-3-yl}ethylamine maleate hydrate;

88) 2-[5-(3-Benzyl-2,5-dioxoimidazolidinylmethyl)-1H-indol-3-yl)ethylamine maleate. 0.5 hydrate, softens 91 °C (dec. 149-151 °C);

89) 2-[5-(3-Benzyl-2-oxoimidazolidinylmethyl)-1H-indol-3-yl]ethylamine maleate.

Pharmaceutical Formulation Examples

In the following Examples, the active compound may be any compound of formula (I) and/or a physiologically acceptable salt or solvate thereof.

(i) Tablet formulations

The following formulations A and B may be prepared by wet granulation of ingredients (a) to (c) and (a) to (d) with a solution of povidone, followed by addition of the magnesium stearate and compression.

Formulation A

| | mg/tablet | mg/tablet |
|---|---|---|
| (a) Active ingredient | 250 | 250 |
| (b) Lactose B.P. | 210 | 26 |
| (c) Sodium Starch Glycollate | 20 | 12 |
| (d) Povidone B.P. | 15 | 9 |
| (e) Magnesium Stearate | 5 | 3 |
| | 500 | 300 |

Formulation B

| | mg/tablet | mg/tablet |
|---|---|---|
| (a) Active ingredient | 250 | 250 |
| (b) Lactose 150 | 150 | - |
| (c) Avicel PH 101 | 60 | 26 |
| (d) Sodium Starch Glycollate | 20 | 12 |
| (e) Povidone B.P. | 15 | 9 |
| (f) Magnesium Stearate | 5 | 3 |
| | 500 | 300 |

Formulation C

| | mg/tablet |
|---|---|
| Active ingredient | 100 |
| Lactose | 200 |
| Starch | 50 |
| Povidone | 5 |
| Magnesium Stearate | 4 |
| | 359 |

The following formulations D and E may be prepared by direct compression of the admixed ingredients. The lactose used in formulation E is of the direct compression type.

Formulation D

| | mg/tablet |
|---|---|
| Active ingredient | 250 |
| Magnesium Stearate | 4 |
| Pregelatinised Starch NF15 | 146 |
| | 400 |

Formulation E

| | mg/tablet |
|---|---|
| Active ingredient | 250 |
| Magnesium Stearate | 5 |
| Lactose | 145 |
| Avicel | 100 |
| | 500 |

Formulation F (Controlled release formulation)

| | mg/tablet |
|---|---|
| (a) Active ingredient | 500 |
| (b) Hydroxypropylmethylcellulose (Methocel K4M Premium) | 112 |
| (c) Lactose B.P. | 53 |
| (d) Povidone B.P.C. | 28 |
| (e) Magnesium Stearate | 7 |
| | 700 |

The formulation may be prepared by wet granulation of ingredients (a) to (c) with a solution of povidone, followed by addition of the magnesium stearate and compression.

(ii) Capsule formulations

Formulation A

Capsules may be prepared by admixing the ingredients of Formulation D above and filling two-part hard gelatin capsules with the resulting mixture. Formulation B (infra) may be prepared in a similar manner.

Formulation B

| | mg/capsule |
|---|---|
| (a) Active ingredient | 250 |
| (b) Lactose B.P. | 143 |
| (c) Sodium Starch Glycollate | 25 |
| (d) Magnesium Stearate | 2 |
| | 420 |

Formulation C

| | mg/capsule |
|---|---|
| (a) Active ingredient | 250 |
| (b) Macrogol 4000 BP | 350 |
| | 600 |

Capsules may be prepared by melting the Macrogol 4000 BP, dispersing the active ingredient in the melt, and filling two-part hard gelatin capsules therewith.

Formulation D

| | mg/capsule |
|---|---|
| Active ingredient | 250 |
| Lecithin | 100 |
| Arachis Oil | 100 |
| | 450 |

Capsules may be prepared by dispersing the active ingredient in the lecithin and arachis oil and filling soft, elastic gelatin capsules with the dispersion.

Formulation E (Controlled release capsule)

| | mg/capsule |
|---|---|
| (a) Active ingredient | 250 |
| (b) Microcrystalline Cellulose | 125 |
| (c) Lactose BP | 125 |
| (d) Ethyl Cellulose | 13 |
| | 513 |

The controlled release capsule formulation may be prepared by extruding mixed ingredients (a) to (c) using an extruder, then spheronising and drying the extrudate. The dried pellets are coated with a release-controlling membrane (d) and filled into two-part, hard gelatin capsules.

(iii) Intravenous injection formulation

| | |
|---|---|
| Active ingredient | 0.200g |
| Sterile, pyrogen-free phosphate buffer (pH 9.0) to | 10 ml |

The active ingredient is dissolved in most of the phosphate buffer at 35-40 °C, then made up to volume and filtered through a sterile micropore filter into sterile 10 ml glass vials (Type 1) which are sealed with sterile closures and overseals.

(iv) Intramuscular injection formulation

| | |
|---|---|
| Active ingredient | 0.20 g |
| Benzyl Alcohol | 0.10 g |
| Glycofurol 75 | 1.45 g |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (Type 1).

(v) Syrup formulation

| | |
|---|---|
| Active ingredient | 0.2500 g |
| Sorbitol Solution | 1.5000 g |
| Glycerol | 1.0000 g |
| Sodium Benzoate | 0.0050 g |
| Flavour | 0.0125 ml |
| Purified Water q.s. to | 5.0 ml |

The sodium benzoate is dissolved in a portion of the purified water and the sorbitol solution added. The active ingredient is added and dissolved. The resulting solution is mixed with the glycerol and then made up to the required volume with the purified water.

(vi) Suppository formulation

| | mg/suppository |
|---|---|
| Active ingredient (63µm)* | 250 |
| Hard Fat, BP (Witepsol H15 - Dynamit NoBel) | 1770 |
| | 2020 |

* The active ingredient is used as a powder wherein at least 90% of the particles are of 63µm diameter or less.

One-fifth of the Witepsol H15 is melted in a steam-jacketed pan at 45°C maximum. The active ingredient is sifted through a 200µm sieve and added to the molten base with mixing, using a Silverson fitted with a cutting head, until a smooth dispersion is achieved. Maintaining the mixture at 45°C, the remaining Witepsol H15 is added to the suspension which is stirred to ensure a homogenous mix. The entire suspension is then passed through a 250µm stainless steel screen and, with continuous stirring, allowed to cool to 40°C. At a temperature of 38-40°C, 2.02g aliquots of the mixture are filled into suitable plastic moulds and the suppositories allowed to cool to room temperature.

(vii) Pessary formulation

| | mg/pessary |
|---|---|
| Active ingredient (63µm) | 250 |
| Anhydrous Dextrose | 380 |
| Potato Starch | 363 |
| Magnesium Stearate | 7 |
| | 1000 |

The above ingredients are mixed directly and pessaries prepared by compression of the resulting mixture.

Biological assay

The compounds of formula (I) prepared in Synthetic Examples 1 to 16 were each tested for their activity as agonists for the "5-$HT_1$-like" receptor mediating smooth muscle contraction by the following method.

Right and left lateral saphenous veins were obtained from male New Zealand White rabbits (2.4-2.7 kg) which had been killed by intravenous injection of pentobarbitone sodium (60 mg/kg). Ring segments (3-5 mm wide) prepared from each vessel were suspended between two wire hooks and immeresed in 20 ml organ baths containing Krebs' solution (pH 7.4) of the following composition (mM): NaCl 118.41, $NaHCO_3$ 25.00, KCl 4.75, $KH_2PO_4$ 1.19, $MgSO_4$ 1.19, glucose 11.10 and $CaCl_2$ 2.50. Cocaine (30µM) was present in

the Krebs' solution throughout the experiment to prevent the uptake of amines by sympathetic neurones. The Krebs' solution was maintained at 37°C and continually gassed with 95% oxygen/5% carbon dioxide. Increases in tissue isometric force were measured using Grass FT03C force displacement transducers and recorded on a Gould BD-212 pen recorder.

A force of 1.0g was applied to each preparation and re-established twice during a subsequent period of 30 minutes. During this period, tissues were exposed to pargyline (500μM) to irreversibly inhibit monoamine oxidase and to phenoxybenzamine (0.1μM) to inactivate $\alpha_1$-adrenoceptors. At the end of the 30 minutes, the inhibitors were removed by several changes of the organ bath Krebs' solution.

Agonist activity was assessed by cumulative additions of the test compound, its concentration being increased in 0.5 $\log_{10}$ unit increments until further additions caused no further change in tissue force. In each experiment, the activity of the test compound was compared to the activity of 5-HT. Activity was expressed in terms of the $p[A_{50}]$ ($-\log_{10}[M]$, where M is the molar concentration of agonist required to produce half the maximum effect). The results obtained are shown in Table 1.

Table 1

| Example | Activity $p[A_{50}]$ |
|---|---|
| 17 | 6.31 |
| 18 | 6.22 |
| 19 | 6.55 |
| 20 | 5.96 |
| 21 | 7.53 |
| 22 | 7.29 |
| 23 | 5.81 |
| 24 | 4.39 |
| 25 | 5.39 |
| 26 | 7.44 |
| 27 | 6.31 |
| 28 | 6.65 |
| 29 | 6.14 |
| 30 | 6.67 |
| 31 | 6.70 |
| 32 | 4.59 |
| 33 | 6.02 |
| 34 | 6.41 |
| 35 | 5.92 |
| 36 | 7.44 |
| 37 | 5.20 |
| 38 | 6.06 |
| 39 | 6.56 |

| | |
|---|---|
| 40 | 6.62 |
| 41 | 6.65 |
| 42 | 6.01 |
| 43 | 6.64 |
| 44 | 6.89 |
| 45 | 6.39 |
| 46 | 5.81 |
| 47 | 7.26 |
| 48 | 5.91 |
| 49 | 6.56 |
| 50 | 6.46 |
| 51 | 6,50 |
| 52 | 5.31 |
| 53 | 7.00 |
| 54 | 5.47 |
| 55 | 5.58 |
| 56 | 6.28 |
| 57 | 5.20 |
| 58 | 6.62 |
| 59 | 6.26 |
| 60 | 5.71 |
| 61 | 6.26 |
| 62 | 5.63 |
| 63 | 6.81 |
| 64 | 4.84 |
| 65 | 6.72 |
| 66 | 6.04 |
| 67 | 6.33 |
| 68 | 6.74 |
| 69 | 6.65 |
| 70 | 6.57 |
| 71 | 6.90 |
| 72 | 6.43 |
| 73 | 69.5 |
| 74 | 6.83 |
| 75 | 6.51 |

| | |
|---|---|
| 76 | 6.45 |
| 77 | 6.50 |
| 78 | 6.31 |
| 79 | 5.81 |
| 80 | 5.93 |
| 81 | 5.63 |
| 82 | 5.19 |
| 83 | 5.50 |
| 84 | 5.30 |
| 85 | 6.24 |
| 86 | 5.55 |
| 87 | 6.03 |
| 88 | 5.28 |

**Claims**

**Claims for the following Contracting States : AT, BE, CH, DE, FR, GB, IT, LI, LU, NL, SE**

1. A compound of formula (I)

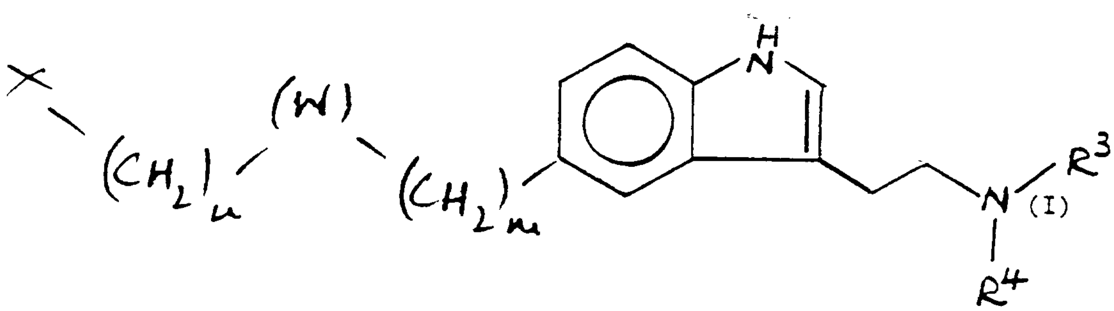

(I)

wherein

$R^3$ and $R^4$ are independently selected from hydrogen, $C_{1-6}$ alkyl (including cycloalkyl) and aryl (wherein the alkyl or aryl group, which latter includes benzyl, is optionally substituted by one or more atoms or groups independently selected from halogen, $C_{1-4}$ alkyl and aryl), provided $R^3 \neq$ benzyl or substituted benzyl when $R^4$ = H;

m is an integer of from 0 to 2;

n is an integer of from 0 to 3;

(W) is a group of formula (i), (ii), (iii), or (iv)

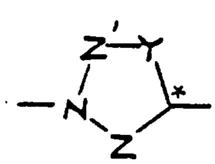

(i)

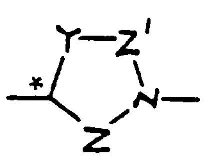

(ii)

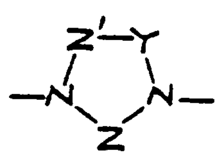

(iii)

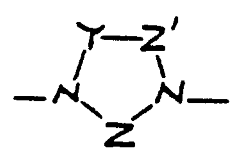

(iv)

wherein Y is selected from oxygen, methylene and >N-$R^5$, where $R^5$ is hydrogen, $C_{1-4}$ alkyl, or benzyl, Z and Z' are independently selected from >C=O, >C=S and methylene, and the chiral centre * in formula (i) or (ii) is in its (S) or (R) form or is a mixture thereof in any proportions;
X is a group selected from
aryl
xanthenyl
dibenzofuranyl
heteroaryl
which group is optionally substituted by an atom or group selected from
aryl
$C_{1-4}$ alkoxy
aryloxy
$C_{1-6}$ alkylsulphonyl
arylsulphonyl
halogen
cyano
carbamoyl
$C_{1-6}$ alkylcarbamoyl
$(C_{1-6})_2$ dialkylcarbamoyl
arylcarbamoyl
$C_{2-7}$ alkanoylamino
aroylamino
$C_{1-6}$ alkylsulphamino
the aryl group(s) of aryl-containing substituents being optionally substituted by halogen or $C_{1-4}$ alkoxy; and salts and solvates thereof.

**2.** A compound of formula (I) as claimed in claim 1, or a salt or solvate thereof, in which (W) is (i) wherein Y = 0, $CH_2$, or $NR^5$, Z = CO or $CH_2$ and Z' = CO, (W) is (ii) wherein Y = $CH_2$ or $NR^5$ and Z = Z' = CO, (W) is (iii) wherein Y = $CH_2$ or $NR^5$, Z = CO and Z' = CO or $CH_2$, or (W) is (iv) wherein Y = $CH_2$, Z = CO and Z' = CO or $CH_2$, and X is a phenyl ring substituted by an alkylcarbamoyl, alkanoylamino, or alkylsulphamino group.

**3.** A compound of formula (I) as claimed in claim 1 or 2, or a salt or solvate thereof, which compound is 2-{5-[1-(3-methylaminocarbonylbenzyl)-2,5-dioxoimidazolidin-4-ylmethyl]-1H-indol-3-yl}ethylamine, N,N-dimethyl-2-[5-{1-[2-(4-acetylaminophenyl)ethyl]-2,5-dioxoimidazolidin-4-ylmethyl}-1H-indol-3-yl]-ethylamine, N-methyl-2-{5-{1-(3-methylaminocarbonylbenzyl)-2,5-dioxoimidazolidin-4-ylmethyl]-1H-indol-3-yl)ethylamine, 2-(5-[1-(3-acetylaminobenzyl)-2,5-dioxoimidazolidin-4-ylmethyl]-1H-indol-3-yl}ethylamine and 2-{5-[1-(3-methylsulphonylaminobenzyl)-2,5-dioxoimidazolidin-4-ylmethyl]-1H-indol-3-yl}ethylamine, or a salt or solvate of any thereof.

**4.** A compound of formula (I) as claimed in any of claims 1 to 3, or a physiologically acceptable salt or solvate thereof, for use in medical therapy.

**5.** A compound of formula (I) as claimed in any of claims 1 to 3, or a physiologically acceptable salt or solvate thereof, for use in the treatment or prophylaxis of a clinical condition in which a selective agonist for the particular type of "5-$HT_1$-like" receptor described herein is indicated.

**6.** A compound of formula (I) as claimed in any of claims 1 to 3, or a physiologically acceptable salt or solvate thereof, for use in the treatment or prophylaxis of a clinical condition in which vasoconstriction in the carotid vascular bed is indicated.

**7.** A compound of formula (I) as claimed in any of claims 1 to 3, or a physiologically acceptable salt or solvate thereof, for use in the treatment or prophylaxis of migraine.

**8.** Use of a compound of formula (I) as claimed in any of claims 1 to 3, or a physiologically acceptable salt or solvate thereof, in the treatment or prophylaxis of a clinical condition as defined in claim 5 or 6.

**9.** Use of a compound of formula (I) as claimed in any of claims 1 to 3, or a physiologically acceptable salt or solvate thereof, in the treatment or prophylaxis of migraine.

**10.** A pharmaceutical formulation comprising a compound of formula (I) as claimed in any of claims 1 to 3, or a physiologically acceptable salt or solvate thereof, and an acceptable carrier therefor.

**Claims for the following Contracting States : ES, GR**

**1.** A process for the preparation of a compound of formula (I)

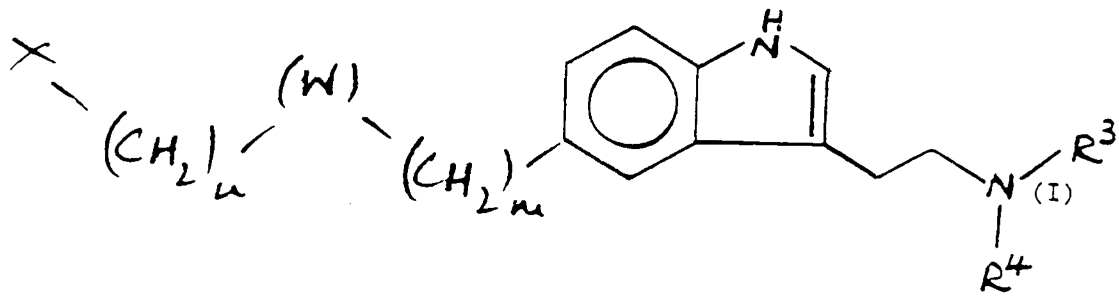

(I)

wherein
$R^3$ and $R^4$ are independently selected from hydrogen, $C_{1-6}$ alkyl (including cycloalkyl) and aryl (wherein the alkyl or aryl group, which latter includes benzyl, is optionally substituted by one or more atoms or groups independently selected from halogen, $C_{1-4}$ alkyl and aryl), provided $R^3 \neq$ benzyl or substituted benzyl when $R^4 = H$;
m is an integer of from 0 to 2;
n is an integer of from 0 to 3;
(W) is a group of formula (i), (ii), (iii), or (iv)

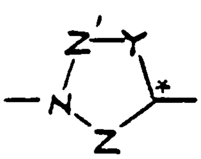

(i)

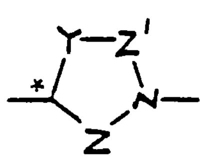

(ii)

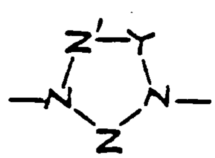

(iii)

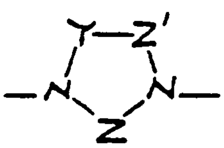

(iv)

wherein Y is selected from oxygen, methylene and $>N-R^5$, where $R^5$ is hydrogen, $C_{1-4}$ alkyl, or benzyl, Z and Z' are independently selected from $>C=O$, $>C=S$ and methylene, and the chiral centre * in formula (i) or (ii) is in its (S) or (R) form or is a mixture thereof in any proportions;
X is a group selected from
- aryl
- xanthenyl
- dibenzofuranyl
- heteroaryl

which group is optionally substituted by an atom or group selected from
- aryl
- $C_{1-4}$ alkoxy
- aryloxy
- $C_{1-6}$ alkylsulphonyl
- arylsulphonyl
- halogen
- cyano

carbamoyl
$C_{1-6}$ alkylcarbamoyl
$(C_{1-6})_2$ dialkylcarbamoyl
arylcarbamoyl
$C_{2-7}$ alkanoylamino
aroylamino
$C_{1-6}$ alkylsulphamino

the aryl group(s) of aryl-containing substituents being optionally substituted by halogen or $C_{1-4}$ alkoxy; and salts and solvates thereof;
the process comprising reacting a compound of formula (II)

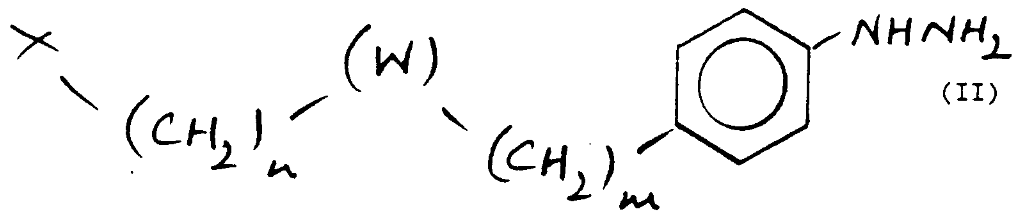

(II)

wherein m, n, (W) and X are as hereinbefore defined, with a compound of formula (III)

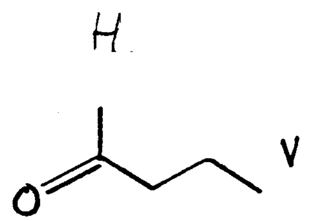

(III)

or a carbonyl-protected form thereof, wherein V is either $-CH_2L$ wherein L is (a) a suitable leaving group and the initial product in aminated, (b) a protected form of amino group and the initial product is deprotected, (c) a nitro group and the initial product is reduced, or (d) a group $-NR^3R^4$ wherein $R^3$ and $R^4$ are as hereinbefore defined other than hydrogen, or V is cyano and the initial product is reduced.

**2.** A process as claimed in claim 1, wherein the compound of formula (II) is prepared by diazotisation of a compound of formula (IV)

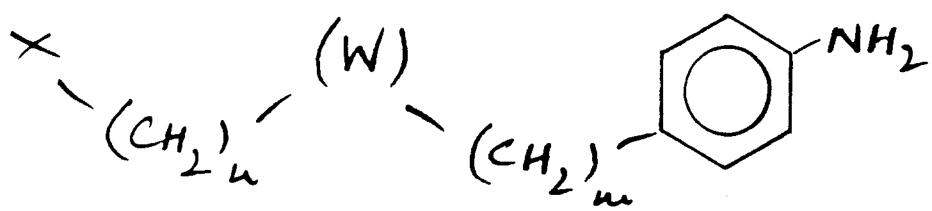

(IV)

wherein m, n, (W) and X are as defined in Claim 1, followed by reduction of the diazo compound.

**3.** A process as claimed in claim 2, wherein the compound of formula (IV) is prepared by reduction of a compound of formula (V)

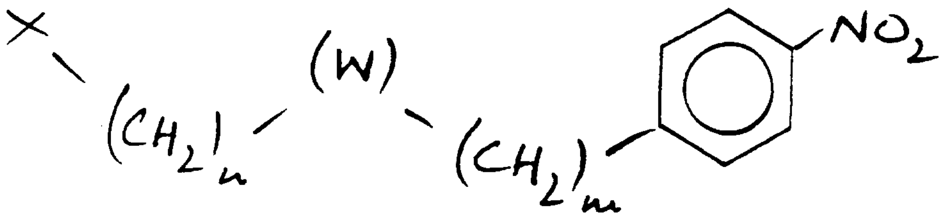 (V)

wherein m, n, (W) and X are as defined in Claim 1.

**4.** A process as claimed in claim 3, wherein the compound of formula (V) is prepared by
(a) reacting a compound of formula (VI)

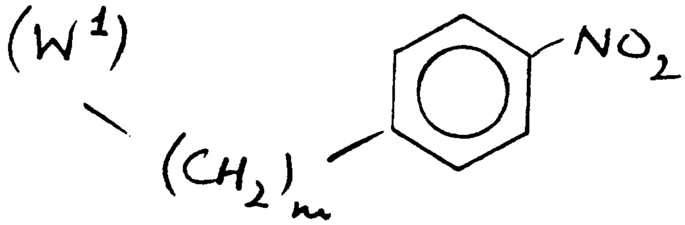 (VI)

wherein m is as defined in Claim 1 and ($W^1$) is

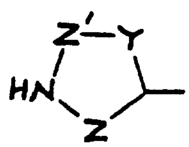 ,

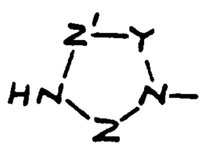 , or 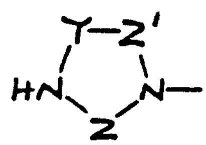 ,

wherein Y, Z and Z' are as defined in Claim 1, with a compound of formula (VII)

$X\text{-}(CH_2)_n\text{-}M$ (VII)

wherein n and X are as defined in Claim 1 and M is a suitable leaving group; or
reacting a compound of formula (VI) wherein m is as hereinbefore defined and ($W^1$) is

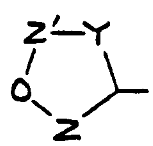 , 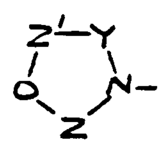 ,

or

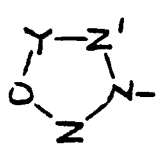 ,

wherein Y, Z and Z' are as hereinbefore defined, with a compound of formula (VII) wherein n and X are as defined in Claim 1 and M is amino;
(b) reacting a compound of formula (VIII)

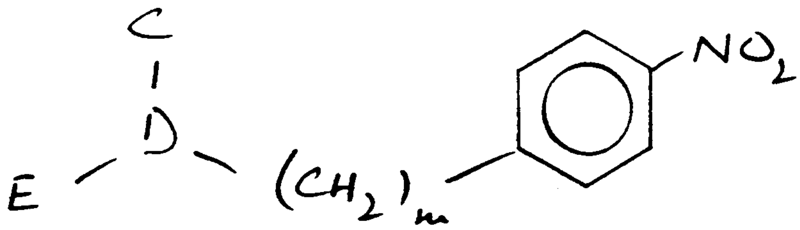

(VIII)

wherein m is as defined in Claim 1, D = CH or N, and C and E are as defined below, with a compound of formula (IX)

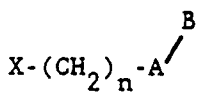

(IX)

wherein n and X are as defined in Claim 1 and A and B are atoms or groups capable or reacting with groups E and C respectively to form a heterocyclic group

$$-(\overline{A-B-C-D-E})-$$

corresponding to a group (W) as defined in Claim 1, or reacting a compound of formula (VIIIA)

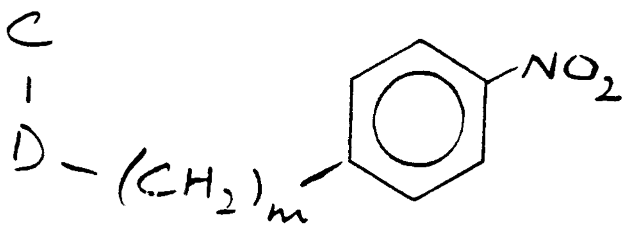

(VIIIA)

wherein m is as defined in Claim 1 and C and D are as defined below, with a compound of formula (IXA)

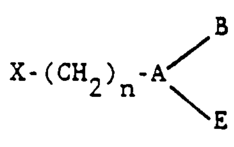

(IXA)

wherein n is as defined in Claim 1, A = CH or N, and B and E are groups capable of reacting with atoms or groups C and D to form a heterocyclic group

$$-(\overline{A-B-C-D-E})-$$

corresponding to a group (W) as defined in Claim 1;

(c) reacting a compound of formula (X)

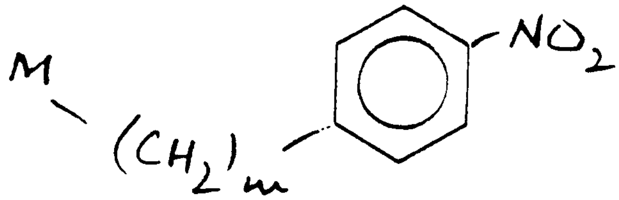

(X)

wherein m is as defined in Claim 1 and M is a suitable leaving group, with a compound of formula (XI)

$X-(CH_2)_n-(W^2)$ (XI)

wherein n and X are as defined in Claim 1 and ($W^2$) is

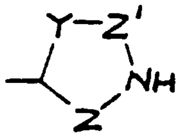 , 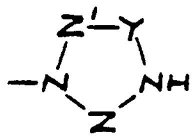 , or 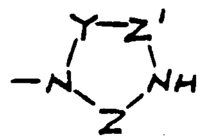 ,

wherein Y, Z and Z' are as defined in Claim 1; or
reacting a compound of formula (X) wherein m is as defined in Claim 1 and M is amino, with a compound of formula (XI) wherein n and X are as hereinbefore defined and ($W^2$) is

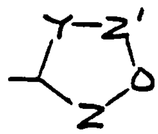 ,

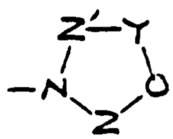 , or 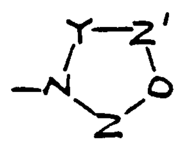 ,

wherein Y, Z and Z' are as hereinbefore defined; or
(d) cyclising a compound of formula (XII)

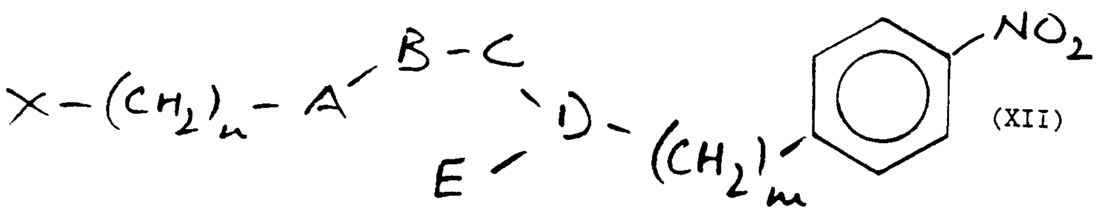

(XII)

or (XIIA)

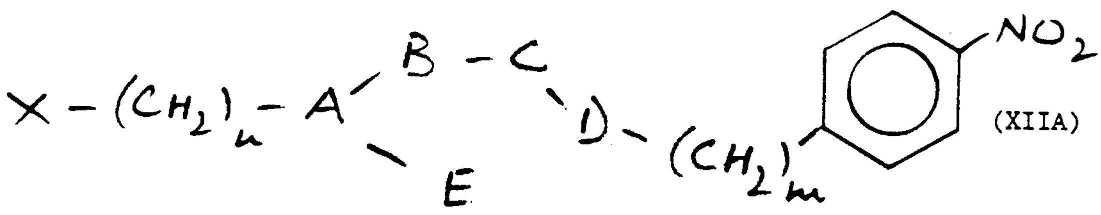

(XIIA)

wherein m, n, A, B, C, D, E and X are as defined in Claim 1.

**5.** A process as claimed in claim 4(a), wherein the compound of formula (VI) is prepared by reacting a compound of formula (XIII)

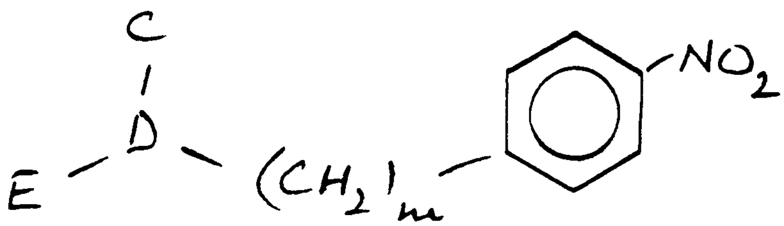

(XIII)

wherein m, C, D and E are as defined in Claim 1, with a compound or anion of formula A-B wherein A and B are as defined in Claim 1, to form a compound of formula (VI).

**6.** A process as claimed in claim 4(a), wherein the compound of formula (VI) is prepared by reacting a compound of formula (X)

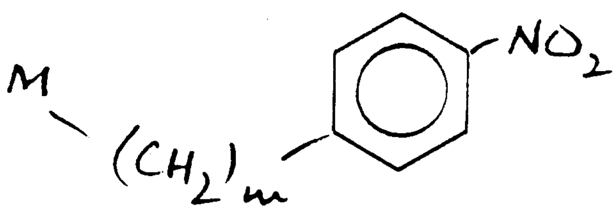

(X)

wherein m and M are as defined in Claim 1, with a compound of formula $H(W^2)$ wherein $(W^2)$ is as defined in Claim 1.

**7.** A process as claimed in claim 4(c), wherein the compound of formula (XI) is prepared by reacting a compound of formula (IXA) wherein n, A, B, E and X are as defined in Claim 1, with a compound or anion of formula C-D wherein C and D are as defined in Claim 1, to form a compound of formula (XI).

**8.** A process as claimed in claim 4(d), wherein the compound of formula (XII) is prepared by reacting a compound of formula (XV)

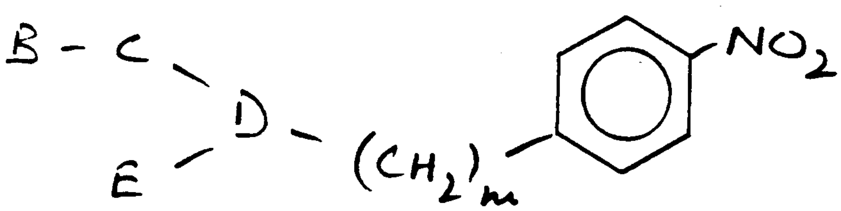

(XV)

wherein m, B, C, D and E are as defined in Claim 1, with a compound of formula (XVI)

$X-(CH_2)_n-A$ (XVI)

wherein n and X are as defined in Claim 1 and A is amino.

**9.** A process as claimed in claim 4(d), wherein the compound of formula (XIIA) is prepared by reacting a compound of formula (XVII)

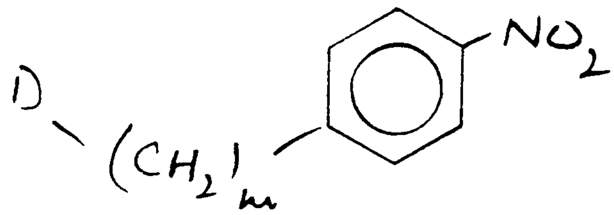

(XVII)

wherein m is as defined in Claim 1 and D is amino, with a compound of formula (XVIII)

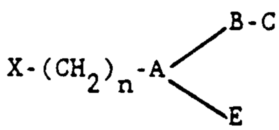

(XVIII)

wherein n, A, B, C, E and X are as defined in Claim 1.

**10.** A process as claimed in claim 4(b), wherein the group -A-B in the compound of formula (IX) is isocyanate.

**11.** A process as claimed in claim 5 or 7, wherein A-B or C-D respectively is cyanate anion.

**Patentansprüche**
**Patentansprüche für folgende Vertragsstaaten : AT, BE, CH, DE, FR, GB, IT, LI, LU, NL, SE**

**1.** Verbindung der Formel (I)

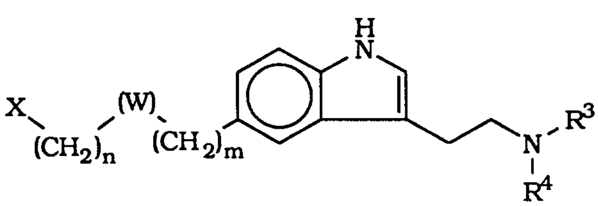

(I)

in der
$R^3$ und $R^4$ unabhängig voneinander ausgewählt sind aus Wasserstoff, $C_{1-6}$-Alkyl (einschließlich Cycloalkyl) und Aryl (worin die Alkyl- oder Arylgruppe, welch letztere Benzyl einschließt, gegebenenfalls durch ein oder mehrere Atome oder Gruppen substituiert ist, die unabhängig voneinander aus Halogen, $C_{1-4}$-Alkyl und Aryl ausgewählt sind), mit der Maßgabe, daß $R^3 \neq$ Benzyl oder substituiertes Benzyl ist, wenn $R^4$ = H bedeutet;
m eine ganze Zahl mit einem Wert von 0 bis 2 darstellt;
n eine ganze Zahl mit einem Wert von 0 bis 3 darstellt;
(W) eine Gruppe der Formel (i), (ii), (iii) oder (iv)

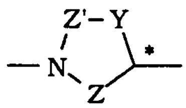 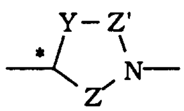 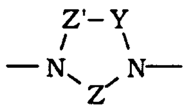 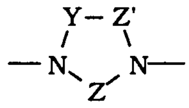

(i) (ii) (iii) (iv)

darstellt, worin Y aus Sauerstoff, Methylen und >N-$R^5$, in der $R^5$ Wasserstoff, $C_{1-4}$-Alkyl oder Benzyl darstellt, ausgewählt ist, Z und Z' unabhängig voneinander aus >C = O, >C = S und Methylen ausgewählt sind, und das chirale Zentrum * in Formel (i) oder (ii) in der (S)- oder (R)-Form oder einer Mischung davon in beliebigen Verhältnissen vorliegt;
X eine Gruppe ausgewählt aus
    Aryl,
    Xanthenyl,
    Dibenzofuranyl,
    Heteroaryl
darstellt, welche Gruppe gegebenenfalls durch ein Atom oder eine Gruppe substituiert ist, ausgewählt aus
    Aryl,
    $C_{1-4}$-Alkoxy,
    Aryloxy,
    $C_{1-6}$-Alkylsulfonyl,
    Arylsulfonyl,
    Halogen,
    Cyano,
    Carbamoyl,
    $C_{1-6}$-Alkylcarbamoyl,
    $(C_{1-6})_2$-Dialkylcarbamoyl,
    Arylcarbamoyl,
    $C_{2-7}$-Alkanoylamino,
    Aroylamino und
    $C_{1-6}$-Alkylsulfamino,
wobei die Arylgruppe(n) der Aryl enthaltenden Substituenten gegebenenfalls durch Halogen oder $C_{1-4}$-Alkoxy substituiert ist;
und deren Salze und Solvate.

**2.** Verbindung der Formel (I) gemäß Anspruch 1, oder ein Salz oder ein Solvat davon, worin (W) (i) bedeutet, worin Y = O, $CH_2$ oder $NR^5$, Z = CO oder $CH_2$ und Z' = CO darstellen, (W) (ii) bedeutet, worin Y = $CH_2$ oder $NR^5$ und Z = Z' = CO darstellen, (W) (iii) bedeutet, worin Y = $CH_2$ oder $NR^5$, Z = CO und Z' = CO oder $CH_2$ darstellen, oder (W) (iv) bedeutet, worin Y = $CH_2$, Z = CO und Z' = CO oder $CH_2$ darstellen, und X einen durch eine Alkylcarbamoyl-, Alkanoylamino- oder Alkylsulfaminogruppe substituierten Phenylring bedeutet.

**3.** Verbindung der Formel (I) nach Anspruch 1 oder 2, oder ein Salz oder ein Solvat davon, nämlich 2-{5-[1-(3-Methylaminocarbonylbenzyl)-2,5-dioxoamidazolidin-4-yl-methyl]-1H-indol-3-yl}ethylamin, N,N-Dimethyl-2-[5-{1-[2-(4-acetylaminophenyl)ethyl]-2,5-dioxoimidazolidin-4-yl-methyl}-1H-indol-3-yl]-ethylamin, N-Methyl-2-{5-[1-(3-methylaminocarbonylbenzyl)-2,5-dioxoimidazolidin-4-yl-methyl]-1H-indol-3yl}ethylamin, 2-{5-[1-(3-Acetylaminobenzyl)-2,5-dioxoimidazolidin-4-yl-methyl]-1H-indol-3-yl}ethylamin und 2-{5-[1-(3-Methylsulfonylaminobenzyl)-2,5-dioxoimidazolidin-4-yl-methyl]-1H-indol-3-yl)ethylamin oder ein Salz oder ein Solvat davon.

**4.** Verbindung der Formel (I) nach einem der Ansprüche 1 bis 3 oder ein physiologisch annehmbares Salz oder Solvat davon zur Verwendung in der medizinischen Therapie.

**5.** Verbindung der Formel (I) nach einem der Ansprüche 1 bis 3 oder ein physiologisch annehmbares Salz oder Solvat davon zur Verwendung bei der Behandlung oder der Prophylaxe eines klinischen Zustands, bei dem ein selektiver Agonist für den hierin beschriebenen speziellen Typus des "5-$HT_1$-artigen"

Rezeptors indiziert ist.

6. Verbindung der Formel (I) nach einem der Ansprüche 1 bis 3 oder ein physiologisch annehmbares Salz oder Solvat davon zur Verwendung bei der Behandlung oder Prophylaxe eines klinischen Zustands, bei dem eine Gefäßverengung im Halsschlagadergefäßbett indiziert ist.

7. Verbindung der Formel (I) nach einem der Ansprüche 1 bis 3 oder ein physiologisch annehmbares Salz oder Solvat davon zur Verwendung bei der Behandlung oder der Prophylaxe von Migräne.

8. Verwendung einer Verbindung der Formel (I) nach einem der Ansprüche 1 bis 3 oder eines physiologisch annehmbaren Salzes oder Solvats davon bei der Behandlung oder der Prophylaxe eines klinischen Zustands, wie er in Anspruch 5 oder 6 definiert ist.

9. Verwendung einer Verbindung der Formel (I) nach einem der Ansprüche 1 bis 3 oder eines physiologisch annehmbaren Salzes oder Solvats davon bei der Behandlung oder Prophylaxe von Migräne.

10. Pharmazeutische Zubereitung enthaltend eine Verbindung der Formel (I) nach einem der Ansprüche 1 bis 3 oder ein physiologisch annehmbares Salz oder Solvat davon und einen dafür geeigneten Träger.

**Patentansprüche für folgende Vertragsstaaten : ES, GR**

1. Verfahren zur Herstellung einer Verbindung der Formel (I)

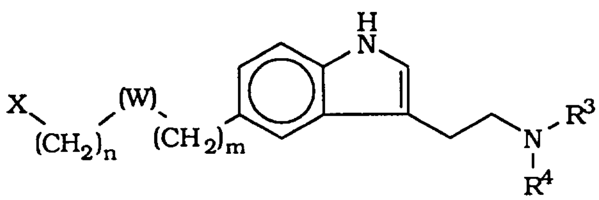

(I)

in der
$R^3$ und $R^4$ unabhängig voneinander ausgewählt sind aus Wasserstoff, $C_{1-6}$-Alkyl (einschließlich Cycloalkyl) und Aryl (worin die Alkyl- oder Arylgruppe, welch letztere Benzyl einschließt, gegebenenfalls durch ein oder mehrere Atome oder Gruppen substituiert ist, die unabhängig voneinander aus Halogen, $C_{1-4}$-Alkyl und Aryl ausgewählt sind), mit der Maßgabe, daß $R^3 \neq$ Benzyl oder substituiertes Benzyl ist, wenn $R^4$ = H bedeutet;
m eine ganze Zahl mit einem Wert von 0 bis 2 darstellt;
n eine ganze Zahl mit einem Wert von 0 bis 3 darstellt;
(W) eine Gruppe der Formel (i), (ii), (iii) oder (iv)

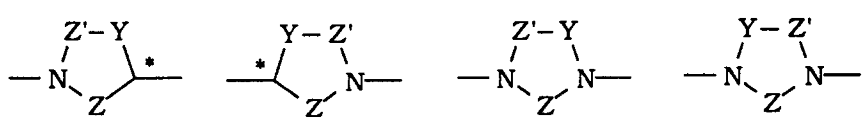

(i) (ii) (iii) (iv)

darstellt, worin Y aus Sauerstoff, Methylen und $>N-R^5$, in der $R^5$ Wasserstoff, $C_{1-4}$-Alkyl oder Benzyl darstellt, ausgewählt ist, Z und Z' unabhängig voneinander aus $>C=O$, $>C=S$ und Methylen ausgewählt sind, und das chirale Zentrum * in Formel (i) oder (ii) in der (S)- oder (R)-Form oder einer Mischung davon in beliebigen Verhältnissen vorliegt;
X eine Gruppe ausgewählt aus
Aryl,
Xanthenyl,
Dibenzofuranyl,

Heteroaryl

darstellt, welche Gruppe gegebenenfalls durch ein Atom oder eine Gruppe substituiert ist, ausgewählt aus

Aryl,
$C_{1-4}$-Alkoxy,
Aryloxy,
$C_{1-6}$-Alkylsulfonyl,
Arylsulfonyl,
Halogen,
Cyano,
Carbamoyl,
$C_{1-6}$-Alkylcarbamoyl,
$(C_{1-6})_2$-Dialkylcarbamoyl,
Arylcarbamoyl,
$C_{2-7}$-Alkanoylamino,
Aroylamino und
$C_{1-6}$-Alkylsulfamino,

wobei die Arylgruppe(n) der Aryl enthaltenden Substituenten gegebenenfalls durch Halogen oder $C_{1-4}$-Alkoxy substituiert ist;
und Salzen und Solvaten davon,
welches Verfahren darin besteht, daß man eine Verbindung der Formel (II)

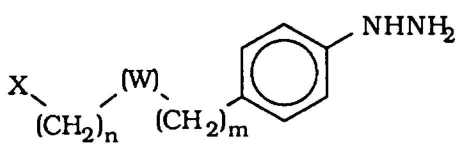

(II)

in der m, n (W) und X die oben angegebenen Bedeutungen besitzen, mit einer Verbindung der Formel (III)

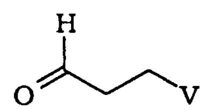

(III)

oder einer an der Carbonylgruppe geschützten Form davon umsetzt, worin V entweder eine Gruppe der Formel $-CH_2-L$, worin L (a) eine geeignete austretende Gruppe darstellt und das zunächst erhaltene Produkt aminiert wird, (b) eine geschützte Form einer Aminogruppe darstellt und die Schutzgruppe von dem zunächst erhaltenen Produkt abgespalten wird, (c) eine Nitrogruppe darstellt und das zunächst erhaltene Produkt reduziert wird oder (d) eine Gruppe der Formel $-NR_3R^4$ darstellt, worin $R^3$ und $R^4$ die oben angegebenen Bedeutungen mit Ausnahme von Wasserstoff besitzen, oder V eine Cyanogruppe darstellt und das zunächst erhaltene Produkt reduziert wird.

**2.** Verfahren nach Anspruch 1, worin die Verbindung der Formel (II) durch Diazotieren einer Verbindung der Formel (IV)

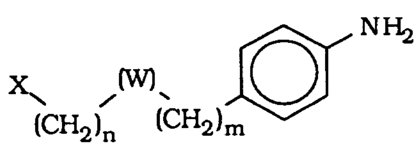

(IV)

in der m, n, (W) und X die in Anspruch 1 angegebenen Bedeutungen besitzen, gefolgt von einer

Reduktion der Diazoverbindung hergestellt wird.

**3.** Verfahren nach Anspruch 2, worin die Verbindung der Formel (IV) durch Reduktion einer Verbindung der Formel (V)

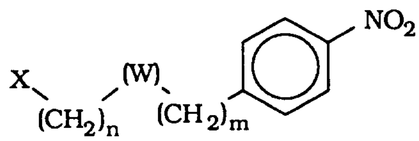

(V)

in der m, n, (W) und X die in Anspruch 1 angegebenen Bedeutungen besitzen, hergestellt wird.

**4.** Verfahren nach Anspruch 3, worin die Verbindung der Formel (V) dadurch hergestellt wird, daß man
(a) eine Verbindung der Formel (VI)

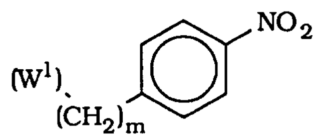

(VI)

in der m die in Anspruch 1 angegebenen Bedeutungen besitzt und ($W^1$) eine Gruppe der Formel

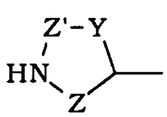
, 
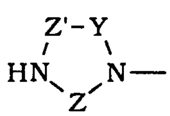
, oder
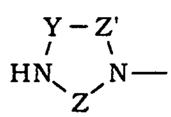

worin Y, Z und Z' die in Anspruch 1 angegebenen Bedeutungen besitzen, darstellt, mit einer Verbindung der Formel (VII)

$X - (CH_2)_n$-M (VII)

in der n und X die in Anspruch 1 angegebenen Bedeutungen besitzen und M eine geeignete austretende Gruppe darstellt, umsetzt; oder
eine Verbindung der Formel (VI), in der m die oben angegebenen Bedeutungen besitzt und ($W^1$) eine Gruppe der Formel

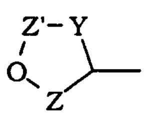
, 
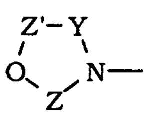
, oder
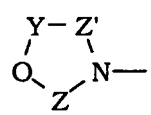

in der Y, Z und Z' die oben angegebenen Bedeutungen besitzen, darstellt, mit einer Verbindung der Formel (VII), in der n und X die in Anspruch 1 angegebenen Bedeutungen besitzen und M eine Aminogruppe darstellt, umsetzt;
(b) eine Verbindung der Formel (VIII)

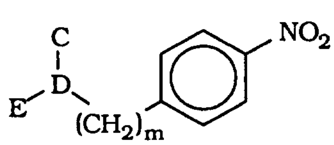

(VIII)

in der m die in Anspruch 1 angegebenen Bedeutungen besitzt, D CH oder N darstellt und C und E die nachfolgend angegebenen Bedeutungen besitzen, mit einer Verbindung der Formel (IX)

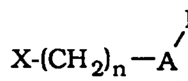

(IX)

in der n und X die in Anspruch 1 angegebenen Bedeutungen besitzen und A und B Atome oder Gruppen darstellen, die dazu geeignet sind, mit Gruppen E bzw. C zu reagieren unter Bildung einer heterocyclischen Gruppe

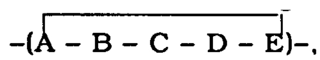

die einer in Anspruch 1 definierten Gruppe (W) entspricht, umsetzt, oder
eine Verbindung der Formel (VIIIA)

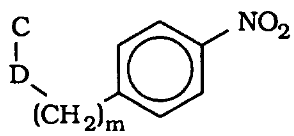

(VIIIA)

in der m die in Anspruch 1 angegebenen Bedeutungen besitzt und C und D die nachfolgend angegebenen Bedeutungen besitzen, mit einer Verbindung der Formel (IXA)

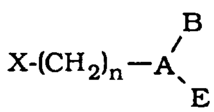

(IXA)

in der n die in Anspruch 1 angegebenen Bedeutungen besitzt, A CH oder N darstellt und B und E Gruppen darstellen, die dazu geeignet sind, mit Atomen oder Gruppen C und D unter Bildung einer heterocyclischen Gruppe

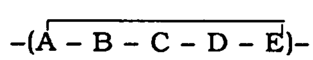

zu reagieren, die einer in Anspruch 1 definierten Gruppe (W) entspricht, umsetzt;
(c) eine Verbindung der Formel (X)

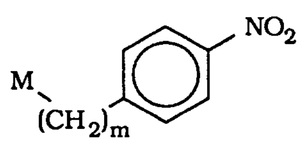

(X)

in der m die in Anspruch 1 angegebenen Bedeutungen besitzt und M eine geeignete austretende Gruppe darstellt, mit einer Verbindung der Formel (XI)

$X - (CH_2)_n-(W^2)$ (XI)

in der n und X die in Anspruch 1 angegebenen Bedeutungen besitzen und $(W^2)$ eine Gruppe der

Formel

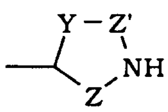 , 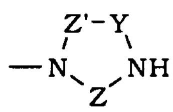 , oder 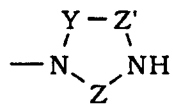

worin Y, Z und Z' die in Anspruch 1 angegebenen Bedeutungen besitzen, darstellt, umsetzt; oder eine Verbindung der Formel (X), worin m die in Anspruch 1 angegebenen Bedeutungen besitzt und M eine Aminogruppe darstellt, mit einer Verbindung der Formel (XI), worin n und X die oben angegebenen Bedeutungen besitzen und ($W^2$) eine Gruppe der Formel

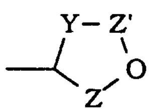 , 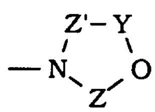 , oder 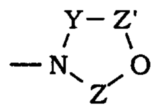

worin Y, Z und Z' die oben angegebenen Bedeutungen besitzen, darstellt, umsetzt; oder
(d) eine Verbindung der Formel (XII)

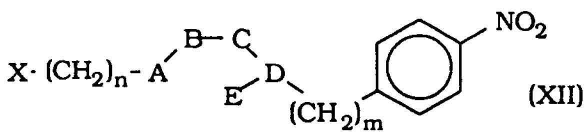

(XII)

oder der Formel (XIIA)

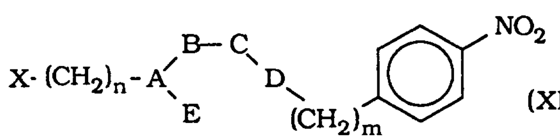

(XIIA)

worinm, n, A, B, C, D, E und X die in Anspruch 1 angegebenen Bedeutungen besitzen, cyclisiert.

**5.** Verfahren nach Anspruch 4(a), worin die Verbindung der Formel (VI) dadurch hergestellt wird, daß man eine Verbindung der Formel (XIII)

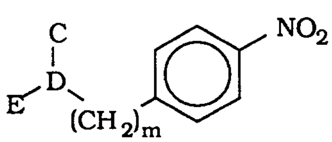

(XIII)

in der m, C, D und E die in Anspruch 1 angegebenen Bedeutungen besitzen, mit einer Verbindung oder einem Anion der Formel A - B, worin A und B die in Anspruch 1 angegebenen Bedeutungen besitzen, unter Bildung einer Verbindung der Formel (VI) umsetzt.

**6.** Verfahren nach Anspruch 4(a), worin die Verbindung der Formel (VI) dadurch hergestellt wird, daß man eine Verbindung der Formel (X)

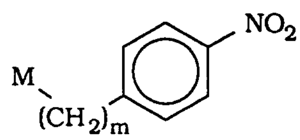

(X)

in der m und M die in Anspruch 1 angegebenen Bedeutungen besitzen, mit einer Verbindung der Formel H(W[2]), worin (W[2]) die in Anspruch 1 angegebenen Bedeutungen besitzt, umsetzt.

**7.** Verfahren nach Anspruch 4(c), worin die Verbindung der Formel (XI) dadurch hergestellt wird, daß man eine Verbindung der Formel (IXA), worin n, A, B, E und X die in Anspruch 1 angegebenen Bedeutungen besitzen, mit einer Verbindung oder einem Anion der Formel C - D, worin C und D die in Anspruch 1 angegebenen Bedeutungen besitzen, unter Bildung einer Verbindung der Formel (XI) umsetzt.

**8.** Verfahren nach Anspruch 4(d), worin die Verbindung der Formel (XII) dadurch hergestellt wird, daß man eine Verbindung der Formel (XV)

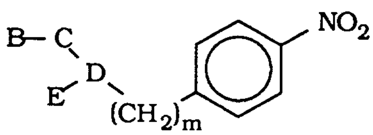

(XV)

in der m, B, C, D und E die in Anspruch 1 angegebenen Bedeutungen besitzen, mit einer Verbindung der Formel (XVI)

$X - (CH_2)_n\text{-}A$ (XVI)

in der n und X die in Anspruch 1 angegebenen Bedeutungen besitzen und A eine Aminogruppe darstellt, umsetzt.

**9.** Verfahren nach Anspruch 4(d), worin die Verbindung der Formel (XIIA) dadurch hergestellt wird, daß man eine Verbindung der Formel (XVII)

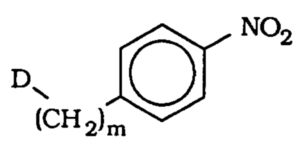

(XVII)

in der m die in Anspruch 1 angegebenen Bedeutungen besitzt und D eine Aminogruppe darstellt, mit einer Verbindung der Formel (XVIII)

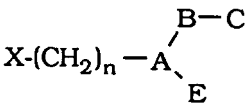

(XVIII)

in der n, A, B, C, E und X die in Anspruch 1 angegebenen Bedeutungen besitzen, umsetzt.

**10.** Verfahren nach Anspruch 4(b), worin die Gruppe -A - B in der Verbindung der Formel (IX) eine Isocyanatgruppe ist.

**11.** Verfahren nach Anspruch 5 oder 7, worin A-B bzw. C - D das Cyanatanion bedeuten.

**Revendications**

**Revendications pour les Etats contractants suivants : AT, BE, CH, DE, FR, GB, IT, LI, LU, NL, SE**

**1.** Composé de formule I

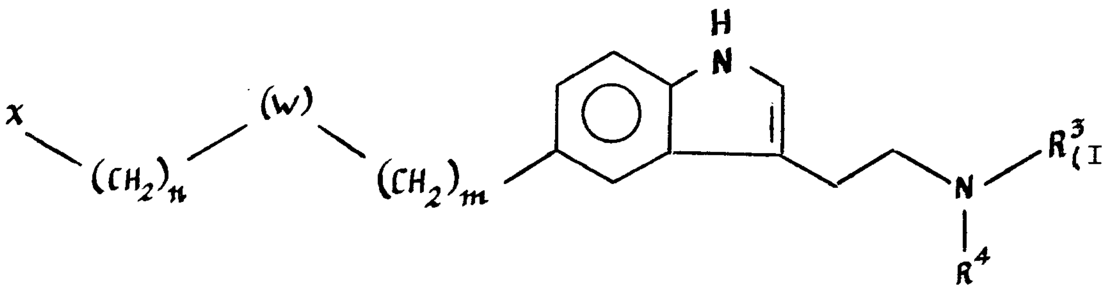

(I)

où $R^3$ et $R^4$ sont choisis indépendamment parmi hydrogène, $C_{1-6}$ alcoyle (y compris cycloalcoyle) et aryle (dont le radical alcoyle ou aryle, lequel dernier inclut benzyle, est facultativement substitué par un ou plusieurs atomes ou radicaux choisis indépendamment parmi halogène, $C_{1-4}$ alcoyle et aryle) avec la restriction que $R^3 \neq$ benzyle ou benzyle substitué lorsque $R^4 = H$;

m est un entier de 0 à 2;

n est un entier de 0 à 3;

(W) est un radical de formule i, ii, iii ou iv

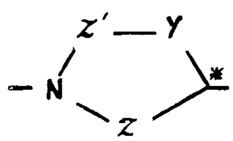

(i)

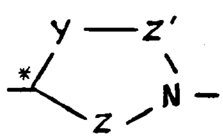

(ii)

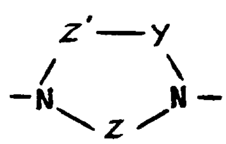

(iii)

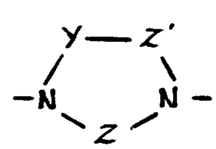

(iv)

où Y est choisi parmi oxygène, méthylène et $>N-R^5$, où $R^5$ est hydrogène, $C_{1-4}$ alcoyle ou benzyle, Z et Z' sont choisis indépendamment parmi $>C=O$, $>C=S$ et méthylène, et le centre chiral * dans la formule i ou ii se trouve sous sa forme (S) ou (R) ou est un mélange de celles-ci en proportions quelconques;

X est un radical choisi parmi :

xanthényle

dibenzofuranyle

hétéroaryle

lequel radical est facultativement substitué par un atome ou radical choisi parmi :

$C_{1-4}$ alcoxy

aryloxy

$C_{1-6}$ alcoylsulfonyle

arylsulfonyle

halogène

cyano

carbamoyle

$C_{1-6}$ alcoylcarbamoyle

$(C_{1-6})_2$ dialcarbamoyle

arylcarbamoyle

$C_{2-7}$ alcanoylamino

aroylamino

$C_{1-6}$ alcoylsulfamino

le ou les radicaux aryle des substituants contenant aryle étant facultativement substitués par halogène ou $C_{1-4}$ alcoxy; et les sels et solvates de celui-ci.

2. Composé de formule I suivant la revendication 1, ou sel ou solvate de celui-ci, dans lequel (W) est (i) où Y = O, $CH_2$ ou $NR^5$, Z = CO ou $CH_2$ et Z' = CO, (W) est (ii) où Y = $CH_2$ ou $NR^5$ et Z = Z' CO, (W) est (iii) où Y = $CH_2$ ou $NR^5$, Z = CO et Z' = CO ou $CH_2$ ou (W) est (iv) où Y = $CH_2$, Z = CO et Z' = CO ou $CH_2$ et X est un cycle phényle substitué par un radical alcoylcarbamoyle, alcanoylamino ou alcoylsulfamino.

3. Composé de formule I suivant la revendication 1 ou 2, ou sel ou solvate de celui-ci, lequel composé est la 2-{5-[1-(3-méthylamino-carbonylbenzyl)-2,5-dioxoimidazolidine-4-ylméthyl]-1H-indole-3-yl}éthylamine, la N,N-diméthyl-2-{5-[1-(2-(4-acétylaminophényl)-éthyl)-2,5-dioxoimidazoline-4-yl-méthyl]-1H-indole-3-yl}éthylamine, la N-méthyl-2-{5-[1-(3-méthylaminocarbonylbenzyl)-2,5-dioxoimidazolidine-4-ylméthyl]-1H-indole-3-yl}éthylamine, la 2-{5-[1-(3-acétylaminobenzyl)-2,5-dioxoimidazolidine-4-ylméthyl]-1H-indole-3-yl}éthylamine ou la 2-{5-[1-(3-méthylsulfonylaminobenzyl)-2,5-dioxoimidazolidine-4-ylméthyl]-1H-indole-3-yl}éthylamine, ou un sel ou solvate de l'une quelconque de celle-ci.

4. Composé de formule I suivant l'une quelconque des revendications 1 à 3, ou sel ou solvate physiologiquement acceptable de celui-ci, à utiliser en thérapeutique médicale.

5. Composé de formule I suivant l'une quelconque des revendications 1 à 3, ou sel ou solvate physiologiquement acceptable de celui-ci, à utiliser pour le traitement ou la prophylaxie d'un état clinique dans lequel un agoniste sélectif pour le type particulier de récepteur "5-$HT_1$-oïde" décrit ici est indiqué.

6. Composé de formule I suivant l'une quelconque des revendications 1 à 3, ou sel ou solvate physiologiquement acceptable de celui-ci, à utiliser dans le traitement ou la prophylaxie d'un état clinique dans lequel une vasoconstriction du lit vasculaire carotidien est indiquée.

7. Composé de formule I suivant l'une quelconque des revendications 1 à 3, ou sel ou solvate physiologiquement acceptable de celui-ci, à utiliser dans le traitement ou la prophylaxie de la migraine.

8. Utilisation d'un composé de formule I suivant l'une quelconque des revendications 1 à 3, ou d'un sel ou solvate physiologiquement acceptable de celui-ci, dans le traitement ou la prophylaxie d'un état clinique tel que défini dans la revendication 5 ou 6.

9. Utilisation d'un composé de formule I suivant l'une quelconque des revendications 1 à 3, ou d'un sel ou solvate physioloqiquement acceptable de celui-ci, dans le traitement ou la prophylaxie de la migraine.

10. Composition pharmaceutique comprenant un composé de formule I suivant l'une quelconque des revendications 1 à 3, ou un sel ou solvate physiologiquement acceptable de celui-ci, ainsi qu'un excipient acceptable.

**Revendications pour les Etats contractants suivants : ES, GR**

1. Procédé pour préparer un composé de formule I

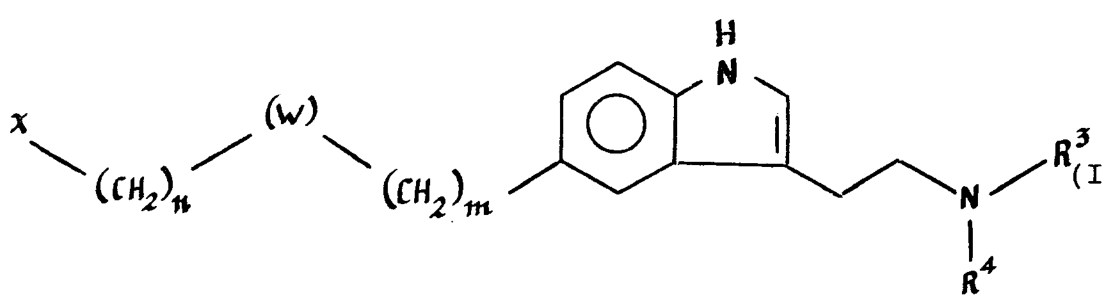

(I)

où $R^3$ et $R^4$ sont choisis indépendamment parmi hydrogène, $C_{1-6}$ alcoyle (y compris cycloalcoyle) et aryle (dont le radical alcoyle ou aryle, lequel dernier inclut benzyle, est facultativement substitué par un ou plusieurs atomes ou radicaux choisis indépendamment parmi halogène, $C_{1-4}$ alcoyle et aryle) avec la restriction que $R^3 \neq$ benzyle ou benzyle substitué lorsque $R^4 = H$;

m est un entier de 0 à 2;

n est un entier de 0 à 3;

(W) est un radical de formule i, ii, iii ou iv

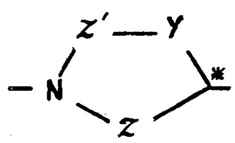

(i)

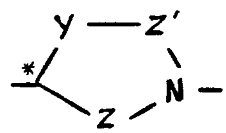

(ii)

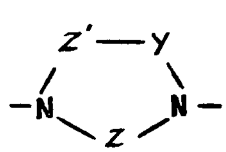

(iii)

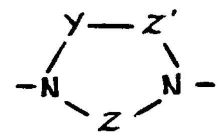

(iv)

où Y est choisi parmi oxygène, méthylène et >N-$R^5$, où $R^5$ est hydrogène, $C_{1-4}$ alcoyle ou benzyle, Z et Z' sont choisis indépendamment parmi >C=O, >C=S et méthylène, et le centre chiral * dans la formule i ou ii se trouve sous sa forme (S) ou (R) ou est un mélange de celles-ci en proportions quelconques;

X est un radical choisi parmi :

xanthényle

dibenzofuranyle

hétéroaryle

lequel radical est facultativement substitué par un atome ou radical choisi parmi :

$C_{1-4}$ alcoxy

aryloxy

$C_{1-6}$ alcoylsulfonyle

arylsulfonyle

halogène

cyano

carbamoyle

$C_{1-6}$ alcoylcarbamoyle

$(C_{1-6})_2$ dialcarbamoyle

arylcarbamoyle

$C_{2-7}$ alcanoylamino

aroylamino

$C_{1-6}$ alcoylsulfamino

le ou les radicaux aryle des substituants contenant aryle étant facultativement substitués par halogène ou $C_{1-4}$ alcoxy; et les sels et solvates de celui-ci,

lequel procédé comprend la réaction d'un composé de formule II

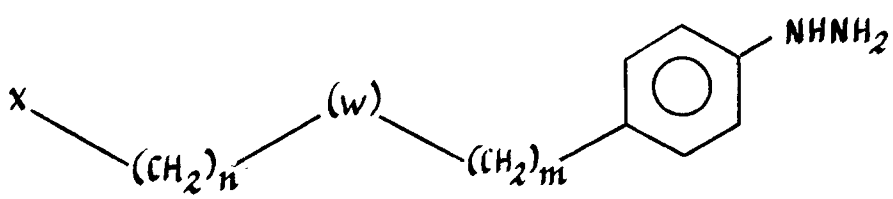

(II)

où n, m, (W) et X sont tels que définis ci-dessus, avec un composé de formule III

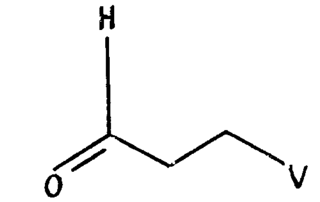

(III)

ou une forme à radical carbonyle protégé de celui-ci, où V est $CH_2L$ où L est (a) un radical partant approprié et le produit initial est aminé, (b) une forme protégée de radical amino et le produit initial est déprotégé, (c) un radical nitro et le produit initial est réduit ou (d) un radical $-NR^3R^4$ où $R^3$ et $R^4$ sont tels que définis ci-dessus autres que d'hydrogène, ou V est cyano et le produit initial est réduit.

**2.** Procédé suivant la revendication 1, dans lequel le composé de formule II est préparé par diazotation d'un composé de formule IV

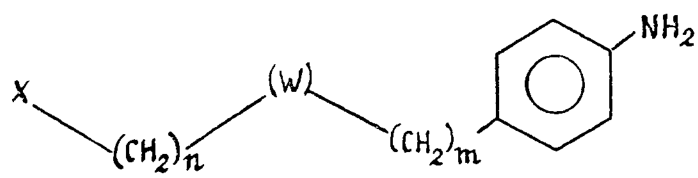

(IV)

où m, n, (W) et X sont tels que définis dans la revendication 1, puis par réduction du composé diazoïque.

**3.** Procédé suivant la revendication 2, dans lequel le composé de formule IV est préparé par réduction d'un composé de formule V

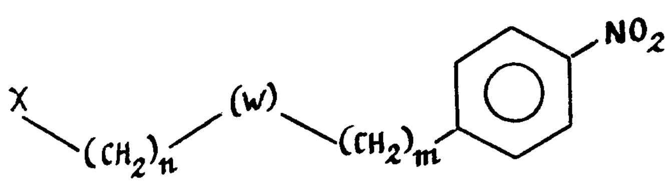

(V)

où m, n, (W) et X sont tels que définis dans la revendication 1.

**4.** Procédé suivant la revendication 3, dans lequel le composé de formule V est préparé par
(a) réaction d'un composé de formule VI

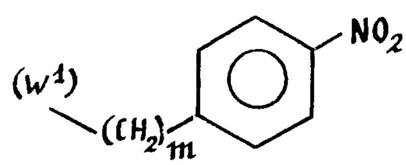

(VI)

où m est tel que défini dans la revendication 1 et
($W^1$) est

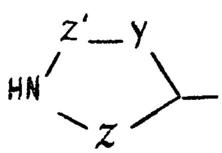 , 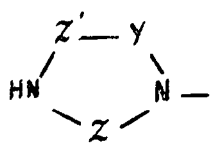 ou 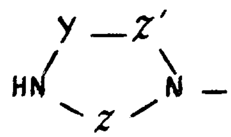 ,

où Y, Z et Z' tels que définis dans la revendication 1,
avec un composé de formule VII

$X\text{-}(CH_2)_n\text{-}M$ (VII)

où n et X sont tels que définis dans la revendication 1 et M est un radical partant approprié; ou réaction d'un composé de formule VI où m est tel que défini ci-dessus et
($W^1$) est

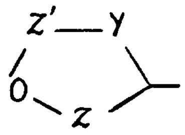
,
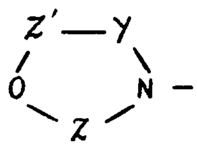
ou
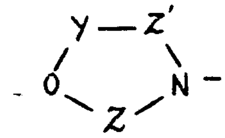
,

où Y, Z et Z' sont tels que définis ci-dessus,
avec un composé de formule VII où n et X sont tels que définis dans la revendication 1 et M est amino;
(b) réaction d'un composé de formule VIII

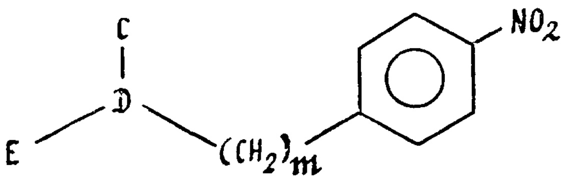
(VIII)

où m est tel que défini dans la revendication 1, D = CH ou N et C et E sont tels que définis ci-après, avec un composé de formule IX

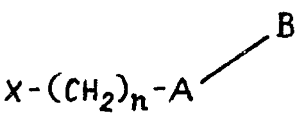
(IX)

où n et X sont tels que définis dans la revendication 1 et A et B sont des atomes ou radicaux capables de réagir avec des radicaux E et C respectivement pour former un radical hétérocyclique

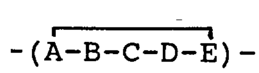

correspondant à un radical (W) tel que défini dans la revendication 1, ou
réaction d'un composé de formule VIIIA

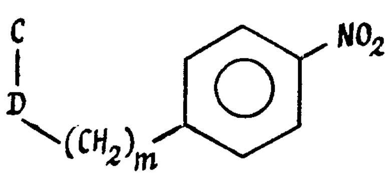
(VIIIA)

où m est tel que défini dans la revendication 1 et C et D sont tels que définis ci-après, avec un composé de formule IXA

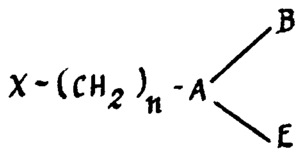

(IXA)

où n est tel que défini dans la revendication 1, A = CH ou N, et B et E sont des radicaux capables de réagir avec des atomes ou radicaux C et D pour former un radical hétérocyclique

$-(\overline{A-B-C-D-E})-$

correspondant à un radical (W) tel que défini dans la revendication 1;
(c) réaction d'un composé de formule X

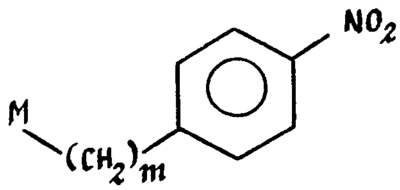

(X)

où m est tel que défini dans la revendication 1 et M est un radical partant approprié, avec un composé de formule XI

$X-(CH_2)_n-(W^2)$ (XI)

où n et X sont tels que définis dans la revendication 1 et ($W_2$) est

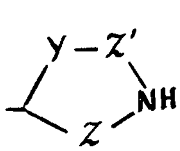 , 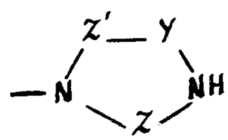 ou 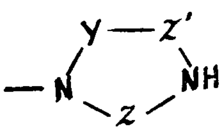 ,

où Y, Z et Z' sont tels que définis dans la revendication 1; ou
réaction d'un composé de formule X où m est tel que défini dans la revendication 1 et M est amino, avec un composé de formule XI où n et X sont tels que définis ci-dessus et ($W^2$) est

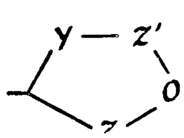 , 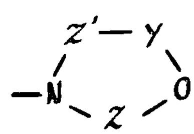 ou 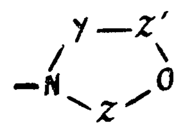

où Y, Z et Z' sont tels que définis ci-dessus; ou
(d) cyclisation d'un composé de formule XII

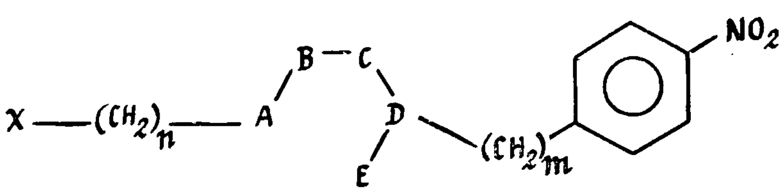

(XII)

ou XIIA

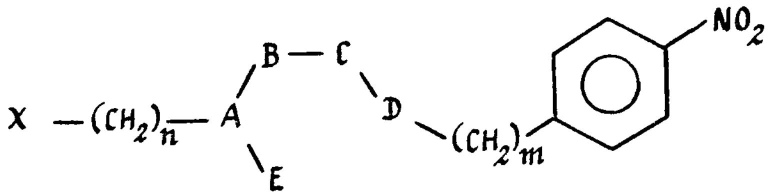

(XIIA)

où m, n, A, B, C, D, E et X sont tels que définis dans la revendication 1.

**5.** Procédé suivant la revendication 4(a), dans lequel le composé de formule VI est préparé par réaction d'un composé de formule XIII

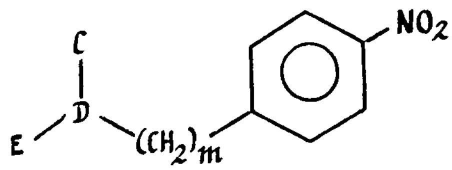

(XIII)

où m, C, D et E sont tels que définis dans la revendication 1, avec un composé ou anion de formule A-B où A et B sont tels que définis dans la revendication 1 pour former un composé de formule VI.

**6.** Procédé suivant la revendication 4(a), dans lequel le composé de formule VI est préparé par réaction d'un composé de formule X

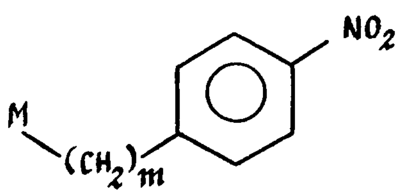

(X)

où m et M sont tels que définis dans la revendication 1, avec un composé de formule $H(W^2)$ où $(W^2)$ est tel que défini dans la revendication 1.

**7.** Procédé suivant la revendication 4(c), dans lequel le composé de formule (XI) est préparé par réaction d'un composé de formule IXA où n, A, B, E et X sont tels que définis dans la revendication 1, avec un composé ou anion de formule C-D où C et D sont tels que définis dans la revendication 1, pour former un composé de formule XI.

**8.** Procédé suivant la revendication 4(d), dans lequel le composé de formule XII est préparé par réaction d'un composé de formule XV

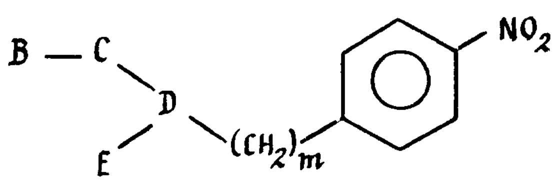

(XV)

où m, B, C, D et E sont tels que définis dans la revendication 1, avec un composé de formule XVI

$X-(CH_2)_n-A$ (XVI)

où n et X sont tels que définis dans la revendication 1 et A est amino.

**9.** Procédé suivant la revendication 4(d), dans lequel le composé de formule XIIA est préparé par réaction d'un composé de formule XVII

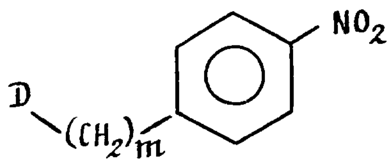

(XVII)

où m est tel que défini dans la revendication 1 et D est amino, avec un composé de formule XVIII

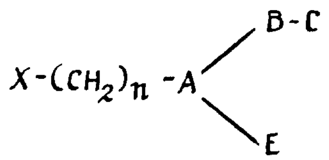

(XVIII)

où n, A, B, C, E et X sont tels que définis dans la revendication 1.

**10.** Procédé suivant la revendication 4(b), dans lequel le radical A-B dans le composé de formule IX est isocyanate.

**11.** Procédé suivant la revendication 5 ou 7, dans lequel A-B ou C-D respectivement est l'anion cyanate.